(19)

**Europäisches Patentamt**
**European Patent Office**
**Office européen des brevets**

(11) **EP 4 137 567 A1**

(12) **EUROPEAN PATENT APPLICATION**

(43) Date of publication:
**22.02.2023 Bulletin 2023/08**

(51) International Patent Classification (IPC):
***C12N 9/02*** (2006.01)          ***C12N 15/63*** (2006.01)
***C12Q 1/66*** (2006.01)

(21) Application number: **21191447.8**

(22) Date of filing: **16.08.2021**

(52) Cooperative Patent Classification (CPC):
**C12N 9/0069**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **Masarykova Univerzita**
  **602 00 BRNO 2 (CZ)**
• **FAKULTNI NEMOCNICE U SV. ANNY V BRNE**
  **65691 Brno (CZ)**

(72) Inventors:
• **Toul, Martin**
  **61500 Brno (CZ)**
• **Schenkmayerová, Andrea**
  **85105 Bratislava (SK)**
• **Marek, Martin**
  **66481 Ostrovacice (CZ)**
• **Rangel Pamplona Pizarro Pinto, Jose Gaspar**
  **60200 Brno (CZ)**

• **Planas Iglesias, Joan**
  **63900 Brno (CZ)**
• **Pluskal, Daniel**
  **67922 Sebrov-Katerina (CZ)**
• **Vasina, Michal**
  **63400 Brno (CZ)**
• **Bednar, David**
  **62500 Brno (CZ)**
• **Prokop, Zbynek**
  **61200 Brno (CZ)**
• **Damborský, Jirí**
  **62800 Brno (CZ)**

(74) Representative: **Dvorakova, Martina**
  **Kania, Sedlak, Smola**
  **Patent Attorneys**
  **Mendlovo namesti 1 a**
  **603 00 Brno (CZ)**

Remarks:
The complete document including Reference Table(s) and the Sequence Listing(s) can be downloaded from the EPO website

(54) **COELENTERAZINE-UTILIZING LUCIFERASES WITH STABLE GLOW-TYPE LIGHT EMISSION, INCREASED SUBSTRATE AFFINITY, SUPPRESED PRODUCT INHIBITION, AND MODULATED EMISSION WAVELENGTHS, AND METHODS OF PRODUCING AND USING THEREOF**

(57)    The present invention includes polynucleotides that encode novel coelenterazine-utilizing luciferases as well as fragments and mutants thereof having at least 85 % sequence identity. The sequences were generated by ancestral sequence reconstruction combined with single-point mutations and transplantation of important regions from the related *Renilla reniformis* luciferase protein. The luciferases according to the present invention exhibit modulated properties including at least one of: increased bioluminescence emission stability and half-life; increased affinity towards substrate over product (suppressed product inhibition); increased thermostability; and modulated emission maximum. The present invention also includes polypeptides that are encoded by the provided polynucleotides as well as fragments and mutants thereof having at least 85 % sequence identity, vectors, kits and expression cassettes containing thereof, cells transfected with thereof, and methods of producing and using thereof.

## Description

Field of the Invention

The present invention relates to modified protein sequences of coelenterazine-utilizing *Renilla-type* luciferases exhibiting stable glow-type light emission, high substrate affinity, low product inhibition factor, and modulated emission maximum, and the production and use of thereof as bioluminescent reporters and bioimaging agents.

Background of the Invention

Luminescence reporters are useful and popular substances in research, allowing easy-to-use, rapid, safe, and sensitive monitoring of biological, biochemical, and chemical processes by measuring an optical signal. The luminescence process is initiated by excitation of luminescent molecules to a high energy state. Return to the ground state with a lower energy is accompanied by release of a photon, observable as light emission. The excitation can be caused by a chemical reaction (chemiluminescence), absorbing photons (photoluminescence, fluorescence, phosphorescence), electric current (electroluminescence) or any other source of energy.

In a bio-based research, bioluminescence reporters are of a particular interest. Bioluminescence is produced by living organisms due to enzymatic reactions catalyzed by proteins denoted as luciferases. The usage of proteins results in a biocompatibility of such luminescence assays, allowing bioimaging and luminescence detection in *vivo* and *in situ.* The first luciferases were isolated from fireflies and click beetles and belong among the most popular bioluminescent reporters until now. However, the larger size of the firefly luciferase and the need for ATP and $Mg^{2+}$ cofactors represent major limitations preventing their usage or making the usage not optimal.

In contrast, coelenterazine-utilizing luciferases are typically small monomeric proteins that do not need any cofactors, and their substrates, coelenterazine and molecular oxygen, are compatible with in *vivo* and *in situ* applications. Coelenterazine (8-benzyl-6-(4-hydroxyphenyl)-2-[(4-hydroxyphenyl)methyl]-7H-imidazo[1,2-a]pyrazin-3-one) is a luciferin molecule that is required for bioluminescence production and that is converted by luciferases found in many aquatic organisms across various phyla. The luciferase RLuc, isolated from the sea pansy *Renilla reniformis,* is the best characterized coelenterazine-utilizing luciferase. RLuc is widely used as a convenient bioluminescent reporter, bioimaging agent, or a fusion tag.

There are, however, limiting factors making the use of RLuc difficult as well. Several attempts to resolve the limitations have been reported. A single-point mutant RLuc-C124A increased the lower serum stability of RLuc more than 6-fold (Liu & Escher, (1999). Gene. 237, 153-159.). An extensive saturation mutagenesis resulted in an eight-point mutant RLuc8 (A55T, C124A, S130A, K136R, A143M, M185V, M253L, and S287L) which exhibited even higher serum stability (more than 220-fold improvement compared to RLuc) but also higher bioluminescence activity (4.3-fold improvement compared to RLuc), and slightly red-shifted emission spectrum with the peak maximum at 487 nm compared to 482 nm for RLuc (Loening et al., (2006). Protein Eng. Des. Sel. 19, 391-400.). Further mutagenesis of RLuc8 resulted in generation of the protein variant RLuc8.6-547 with additional 6 mutations (A123S, D154A, E155G, D162E, I163V, and F262W), yielding a protein with significantly red-shifted emission spectrum with the peak maximum at 547 nm while retaining activity comparable to native RLuc (Loening, Wu & Gambhir, (2007). Nat. Methods. 4, 641-643.). Alternatively, red-shifted bioluminescence spectra can be obtained by using various coelenterazine analogues with emission maxima at wavelengths higher than 520 nm.

Despite many advances and improvements of the original RLuc protein, two major limitations are preserved. First, the flash-type of the bioluminescence signal with short half-life prevents prolonged signal collection and analyses. Second, moderate substrate affinity does not allow analyses at lower coelenterazine concentrations with the maintained level of the detected bioluminescence signal. An attempt to increase the half-life of the bioluminescence was made by generating the recently reported enzyme variant Super RLuc8 (Rahnama el., (2017). Enzyme Microb. Technol. 96, 60-66.) but the improvement was not significant and in contrast, affinity towards the coelenterazine substrate was decreased, making Super RLuc8 inconvenient for practical use, especially at low coelenterazine concentrations. Alternatively, it is possible to use the Dual-Glo Luciferase Assay (Promega, USA) or the *Renilla*-Glo Luciferase Assay System (Promega, USA), increasing the half-life of the bioluminescence signal decay to more than 40 min due to the specific additives. However, these extra chemicals are in contrast with the simple nature of RLuc not requiring cofactors, making these assays unsuitable for certain biological systems or experimental conditions where the additional chemicals cannot be used, e.g., in *vivo,* cell biology, or biomedical applications.

As such, there is a continued need for the development of luciferases that exhibit improved catalytic and spectral properties, namely glow-type bioluminescence signal, suppressed product inhibition, long half-life, and improved substrate affinity saturating enzyme at lower coelenterazine concentrations. The present invention addresses the stated limitations.

Disclosure of the Invention

The present invention was made in view of the prior art described above, and the object of the present invention is to provide novel coelenterazine-utilizing luciferase proteins with improved properties compared to the state-of-the-art solutions that are used in bioluminescence kits as reporters and bioimaging agents. The modulated properties include bioluminescence signal stability, thermostability, substrate affinity, suppressed product inhibition, and emission maximum wavelength.

The limitations of the current coelenterazine-utilizing luciferases were overcome by applying the method of ancestral sequence reconstruction combined with transplantation of dynamic elements important for effective substrate recognition. Ancestral sequence reconstruction generated the most-likelihood sequence of an ancestral protein AncHLD-RLuc (SEQ ID NO: 1) of the *Renilla reniformis* luciferase (RLuc) and sequentially related enzymes haloalkane dehalogenases (Chaloupkova et al., (2019). ACS Catal. 9, 4810-4823.). The ancestral protein possessed minimal bioluminescent luciferase activity but a high thermostability, allowing to accept major modification of the protein backbone structure (see Example 1). Transplantation of the identified dynamic fragments from the *Renilla reniformis* luciferase RLuc into the ancestral protein (see Example 6) resulted in generation novel *Renilla*-type coelenterazine-utilizing luciferases with modulated properties, namely stable glow-type bioluminescence emission, improved affinity towards substrate over product, suppressed product inhibition, and increased thermostability. Further single-point mutagenesis of the newly generated luciferases provided variants with modulated emission maxima of their bioluminescence spectra.

Embodiments of the present invention include polynucleotides that encode protein scaffolds of the ancestral protein (SEQ ID NO: 1) with regions exchanged for the elements of the sequence of the *Renilla reniformis* luciferase RLuc combined with single-point mutations (SEQ ID NO: 2, 3, 4, and 5).

Embodiments of the present disclosure include novel *Renilla-type* coelenterazine-converting luciferase polypeptides (SEQ ID NO: 6, 7, 8, and 9) that are encoded by the provided polynucleotides as well as fragments and mutants thereof having at least 85 % sequence identity to SEQ ID NO: 6, or SEQ ID NO: 7, or SEQ ID NO: 8, or SEQ ID NO: 9 that exhibit modulated properties including at least one of: increased bioluminescence emission stability and half-life; increased affinity towards substrate over product (suppressed product inhibition); increased thermostability; and modulated emission maximum. The preferred is at least 90 %, 95 %, 97 %, or 99 % sequence identity to SEQ ID NO: 6, or SEQ ID NO: 7, or SEQ ID NO: 8, or SEQ ID NO: 9 according to the present invention.

According to the preferred embodiment of the present invention, a coelenterazine-converting polypeptide having at least 85 % sequence identity to SEQ ID NO: 6 or at least 85 % sequence identity to SEQ ID NO: 8 containing at least an amino acid substitution D160A. More preferably the polypeptide has SEQ ID NO: 6 or SEQ ID NO: 8.

The polypeptides according to the present invention differ from the sequence of the AncHLD-RLuc polypeptide (SEQ ID NO: 1) by having DVIESWDEWPDIEEDIALIK in the sequencies thereof (SEQ ID NO: 6 or SEQ ID NO: 7) or by having DVIESWDEWPDIEEAIALIK in the sequences thereof (SEQ ID NO: 8 or SEQ ID NO: 9). Those specific parts of the sequences are in subsequent positions 146 to 165 in the full length polypeptide. It is obvious for a person skilled in the art that positions numbering will change depending on extending or shortening the polypeptide sequence (i.e., insertions and deletions) within the scope of 85 % identity.

According to the preferred embodiment of the present invention, the coelenterazine utilizing polypeptide having at least 85 % sequence identity to SEQ ID NO: 6 containing DVIESWDEWPDIEEDIALIK or at least 85 % sequence identity to SEQ ID NO: 8 containing DVIESWDEWPDIEEAIALIK.

The preferred embodiment of the present invention is a coelenterazine-utilizing polypeptide, wherein a part of SEQ ID NO: 6 or SEQ ID NO: 8 is LVKGGKP. More preferred embodiments of the invention disclose polypeptides wherein the sequences also contain LVKGGKP (SEQ ID NO: 7 and SEQ ID NO: 9). That specific part is in subsequent positions 223 to 229 of sequences SEQ ID NO: 7 or 9 in the full length polypeptide. It is obvious for a person skilled in the art that positions numbering will change depending on extending or shortening the polypeptide sequence (i.e., insertions and deletions) within the scope of 85 % identity.

Embodiments of the present disclosure further include polynucleotides that encode the coelenterazine-utilizing polypeptides according to the present invention. Fragments and mutants thereof having at least 85 % sequence identity to SEQ ID NO: 2, or SEQ ID NO: 3, or SEQ ID NO: 4, or SEQ ID NO: 5 are also included. The preferred is at least 90 %, 95 %, 97 %, or 99 % sequence identity to SEQ ID NO: 2, or SEQ ID NO: 3, or SEQ ID NO: 4, or SEQ ID NO: 5 according to the present invention. The polynucleotide is preferably selected from a group containing SEQ ID NO: 2, SEQ ID NO: 3, SEQ ID NO: 4, or SEQ ID NO: 5.

Embodiments of the present invention also disclose a vector (e.g., a plasmid, a virus, or defective viral particles) containing the polynucleotide sequence according to the present invention, as described above. Embodiments of the invention further include an expression cassette containing the polynucleotide sequence encoding the coelenterazine-utilizing polypeptide according to the present invention. In one embodiment of the present invention, the expression cassette comprises a promoter, e.g., a constitutive or regulatable promoter, operably linked to the polynucleotide sequence according to the present invention. In one embodiment, the expression cassette contains an inducible promoter.

The invention also includes a cell, thus a cell line, or an isolated cell, or a host cell, that expresses the coelenterazine-utilizing polypeptide according to the invention as defined above contains an expression cassette comprising a nucleic acid molecule encoding the coelenterazine-utilizing polypeptide as described above, or contains a vector with the polynucleotide sequence as described above. The origin of the cells can be prokaryotic or eukaryotic, such as plant or vertebrate cells. The vertebrate cells include mammalian cells such as but not limited to human, non-human primate, canine, feline, bovine, equine, ovine, or rodent (e.g., rabbit, rat, ferret, or mouse) cells.

Embodiments of the present invention further disclose a kit, thus a bioluminescence kit, or a gene expression reporting kit, or a bioimaging kit, which comprises the polynucleotide sequence, or the polypeptide sequence, or expression cassette, or vector, or host cell, or modified circularly permuted thermostable luciferase biosensor of the invention.

One embodiment of the present invention comprises a kit containing an expression cassette, or a vector according to the present invention. Such a kit would allow an easy step-by-step transfection of cells or preparation of transgenic organisms that would be expressing the polynucleotides and producing the novel coelenterazine-utilizing luciferases according to the present invention. The kit would thus find a practical use as a standardized material for monitoring the level of gene expression and for bioimaging purposes. By inserting the polynucleotides according to the present invention after the same promoter as the gene of interest, the level of gene expression would be directly proportional to the level of stable bioluminescence (gene expression reporting kit) and the localization of expression in an organism would be easily monitored by stable bioluminescence imaging (bioimaging kit). By joining the polynucleotides according to the present invention with the gene of interest into a single chimeric fusion protein, a monitoring of the produced protein localization would be allowed by in *vivo* stable long-term bioimaging techniques (bioimaging kit).

Another embodiment of the present invention comprises a kit containing a polypeptide according to the present invention. Preferably a bioluminescence kit where, for example, a studied reaction can be coupled to coelenterazine-utilizing luciferase reaction in order to monitor the reaction of interest by a stable bioluminescence signal. The application can include enzyme kinetics for enzymes producing oxygen (e.g., catalase) or chemical kinetics involving superoxide radicals - these components interfere with the luciferase reaction and thus, their production is directly proportional to the intensity of the luminescence signal. Another application covers monitoring effectivity of molecular chaperons (heat shock proteins) in protecting proteins, including the luciferase in a kit according to the present invention, directly proportional to the level of bioluminescence signal.

Embodiments of the present invention also include a polynucleotide encoding a fusion polypeptide composed of a coelenterazine-utilizing polypeptide according to the present invention and at least one fusion partner. By covalently joining a protein of interest with a coelenterazine-utilizing polypeptide, the amount and tissue or cellular localization of the protein of interest would be proportional to the level and position of the stable bioluminescence signal. Fusion of proteins allows for easy monitoring of the protein of interest: its localization, amount, the level of gene expression, and the efficiency of its ribosomal synthesis.

Embodiments of the present invention disclose a method of generation of polynucleotides according to the present invention as well as polypeptides according to the present invention which are encoded by these polynucleotides. The method of generation includes identification of a dynamic region in the state-of-the-art proteins in the field of invention, followed by transplantation of the dynamic region onto a hyperstabilized most likelihood ancestral sequence of the state-of-the-art proteins, resulting in novel sequences with useful modulated properties (see Example 6). Preferably, the method includes transplantation of the dynamic regions helix $\alpha 4$, and/or loop L9, and/or loop L14 of the modern luciferase RLuc protein onto the corresponding positions in the hyperstabilized most likelihood ancestral sequence AncHLD-RLuc (SEQ ID NO: 1) using mutagenic primers and PCR-based mutagenesis.

Embodiments of the present invention further cover a method of producing and isolating polypeptides according to the present invention comprising growing the cell according to the present invention wherein the polynucleotides encoding the polypeptides are expressed, followed by isolating the polypeptides that are substantially free of other proteins. The method of polypeptides production is based on bacterial cells transformation or eukaryotic cells transfection followed by a heterologous overexpression of polynucleotides according to the present invention induced by activating the corresponding promoter. The method of isolating polypeptides according to the present invention is based on a release of overproduced polypeptides from cells by their disruption (e.g., by sonication or mechanical homogenization), followed by a column chromatography purification, separating proteins depending on their different properties, such as hydrophobicity, solubility, molecular weight, or surface charge. More preferred method utilizes affinity tags (e.g., hexahistidine tag, streptavidin tag) covalently linked to the polypeptides according to the present invention for a convenient one-step isolation using affinity chromatography purification, separating polypeptides according to the present invention selectively from other proteins.

Embodiments of the present invention further cover use of polypeptides according to the present invention as bioluminescence reporters or as bioimaging agents.

The novel coelenterazine-utilizing luciferases according to the present invention benefit especially from the glow-type bioluminescence emission, exhibiting a significantly improved signal stability, increased half-life of the signal decay, and suppressed product inhibition (see Examples 7 and 8). These properties are inherent to the luciferases themselves; no additional compounds need to be added. The subjected coelenterazine-utilizing luciferases can be used in research practices where prolonged bioluminescence measurements and long signal stability are essential. Half-lives of the novel coelenterazine-utilizing luciferases (ca 20 minutes) were increased 100-fold compared to the current state-of-the-art enzymes with half-lives of ca 0.2 minutes (see Example 7).

Importantly, the novel coelenterazine-utilizing luciferases according to the present invention exhibit improved preference for the coelenterazine substrate over the enzymatic product (see Example 8 and 12). This property leads to full saturation of the presented luciferases and their maximal catalytic efficiencies at lower coelenterazine concentrations. This property is also the reason of the low product inhibition and prolonged half-life of the bioluminescence signal emission. As a consequence, the subjected coelenterazine-utilizing luciferases can be used in research practices and biomedical applications where minimal coelenterazine concentrations are essential for bioluminescence measurements and where increased coelenterazine concentrations might be toxic and incompatible with a particular application. The effective substrate concentration which yields half of the limiting catalytic rate ($K_m$ constant) of the novel coelenterazine-utilizing luciferases was decreased approximately 50-fold (see Example 8), meaning that 50-fold lower concentration of the substrate is needed to reach the optimal catalytic rates.

Advantageously, the novel coelenterazine-utilizing luciferases according to the present invention exhibit higher thermostabilities compared to other state-of-the-art *Renilla-type* luciferases (see Example 10). This property allows their usage at elevated temperatures and in the assays where preservation of the bioluminescent activity over a long time period is desirable. The values of melting temperatures, defined as temperatures at which half of the molecules are denatured, were increased to 57 °C which is 7-12 °C more than current state-of-the-art solutions.

Some embodiments of the novel coelenterazine-utilizing luciferases according to the present invention will benefit from the modulated bioluminescence emission spectra that were blue-shifted to lower wavelengths (see Example 9). This property is inherent to the modified luciferases themselves; no additional compounds, substrate analogues, or specific solvents need to be added. Crucially, the subjected coelenterazine-utilizing luciferases can be used in research practices and biomedical applications in a combination with the standard *Renilla*-luciferase system to yield a dual-color bioluminescence system while using only a single coelenterazine substrate. Bioluminescence emission maxima of the subjected coelenterazine-utilizing luciferases are up to 90 nm blue-shifted, allowing distinct separation of bioluminescence signals without interfering with each other.

Definitions

The definition of certain terms as used in this present invention are provided below. Unless otherwise defined, all technical and scientific terms used herein have the same meaning.

As used herein, the term "polypeptide" is synonymous with the term "protein".

As used herein, the term "coelenterazine-utilizing luciferase" is synonymous with the terms "coelenterazine-utilizing polypeptide" and "coelenterazine-converting luciferase" and "coelenterazine-converting polypeptide".

As used herein, "modulated properties" refer to at least one improved property of a polypeptide according to the present invention selected from a group comprising: increased bioluminescence emission stability and half-life; increased affinity towards substrate over product; increased thermostability; suppressed product inhibition; and modulated emission maximum.

As used herein, the term "sequence identity" denotes the same amino acid residues are found within any variant of a polypeptide and a polypeptide that serves as a reference when a specified, contiguous segment of a polypeptide amino acid sequence is aligned and compared to another amino acid sequence of a particular corresponding reference molecule. The number of matched positions is calculated by determining the number of positions at which an identical amino acid residue is present in both sequences. Dividing the number of matched positions by the total number of positions in the segment undergoing comparison to the reference molecule, and multiplying the result by 100, yields the percentage of sequence identity. Methods of sequence alignment are well known in the art. The reference sequence used herein refers to a particular corresponding polypeptide of SEQ ID NO: 6, or SEQ ID NO: 7, or SEQ ID NO: 8, or SEQ ID NO: 9 according to the invention. The preferred is at least 85 %, 90 %, 95 %, 97 %, or 99 % sequence identity. A person skilled in the art will understand that the remaining 15 % or fewer amino acids along the length of the polypeptide of SEQ ID NO: 6, or SEQ ID NO: 7, or SEQ ID NO: 8, or SEQ ID NO: 9 according to the invention is variable due to, for example, synonymous or silent effects of particular amino acid residues exchanged at various sequence positions, compensation effects of amino acid residue pairs exchanged at various sequence positions, etc.

Fragments or mutants of the polypeptide with SEQ ID NO: 6, or SEQ ID NO: 7, or SEQ ID NO: 8, or SEQ ID NO: 9 are also included. A person skilled in the art will understand that, for example, the C-terminus or the N-terminus of the polypeptide with SEQ ID NO: 6, or SEQ ID NO: 7, or SEQ ID NO: 8, or SEQ ID NO: 9 can be deleted without losing at least one modulated property as described above.

The term "polynucleotide", as used herein, refers to a nucleic acid molecule, DNA or RNA, that comprises coding sequences necessary for the production of a polypeptide according to the invention. The encoded polypeptide may be a full-length polypeptide, a fragment thereof (shorter than the full-length polypeptide), or a fusion of either the full-length polypeptide or the fragment thereof with another polypeptide, yielding a fusion protein.

As used herein, the term "isolated cell" stands for a specified cell with unique properties, such as inserted foreign genes, altered morphology, or activated signal/metabolic pathways, substantially free of the other living cells.

The terms "cell", "cell line", "host cell", or "isolated cell", as used herein, are used interchangeably, and all such designations include progeny or potential progeny of these designations. The term "transformed cell" refers to a cell into which a nucleic acid molecule of the invention has been introduced. Optionally, a nucleic acid molecule of the invention may be introduced into a suitable cell line so as to create a stable cell line capable of producing the protein or polypeptide encoded by the nucleic acid molecule of the invention. Vectors, cells, and methods for constructing such cell lines are well known in the art. The words "transformants" or "transformed cells" include the primary transformed cells derived from the originally transformed cell with no regard to the number of transfers. All progeny may not be precisely identical in the polynucleotide content, due to deliberate or inadvertent mutations. Nonetheless, mutant progeny, that have the same functionality as screened for in the originally transformed cell, are included in the definition of transformants.

As used herein, the term "fusion protein" refers to a chimeric polypeptide sequence composed of two or more proteins covalently bound together into a single polypeptide with multiple functions of all the original proteins. Fusion proteins are constructed by joining two or more genes originally encoding discrete proteins, and expressing them as a single gene.

As used herein, the term "fusion partner" refers to a protein of interest whose amount, level of gene expression, or localization is proportional to the level of bioluminescence due to its fusion with coelenterazine-utilizing luciferases into a single fusion protein.

The term "vector" refers to nucleic acid molecules into which fragments of polynucleotides may be inserted or cloned. Vectors can be used to transfer polynucleotide segment(s) into a cell where they might be capable of replication. Vectors may be derived from plasmids, bacteriophages, viruses, cosmids, defective viral particles, and the like.

Brief Description of the Drawings

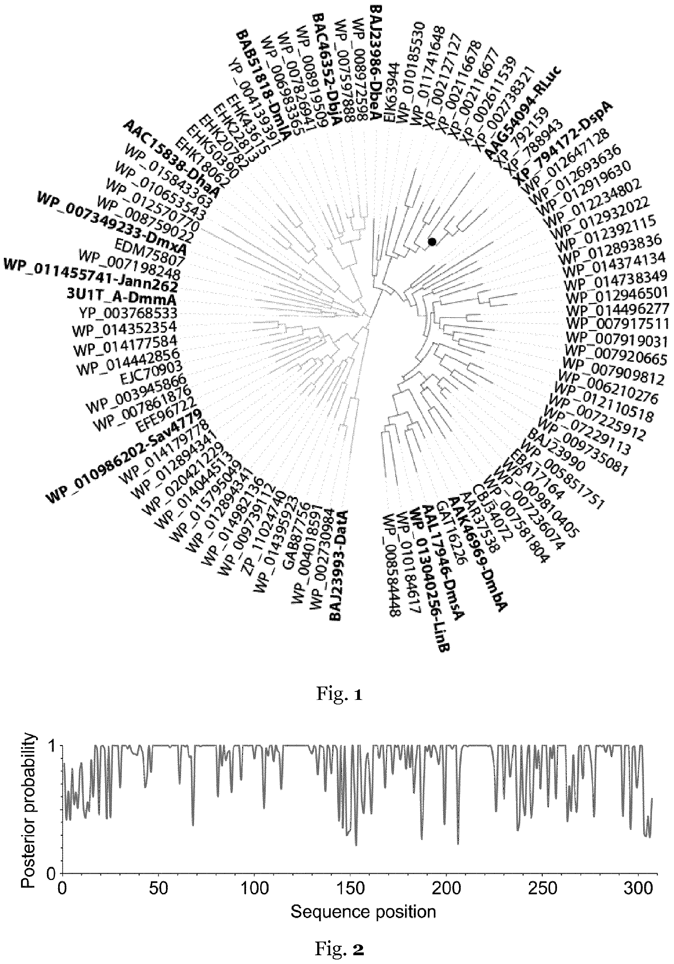

Fig. **1** presents the phylogenetic tree of evolutionarily related haloalkane dehalogenase (subfamily II) and *Renilla* luciferase protein sequences. The position of the reconstructed ancestor AncHLD-RLuc is indicated with the black circle. Experimentally characterized extant enzymes are labeled in bold. Putative dehalogenase enzymes are labeled with their accession numbers. Bootstrap support values are depicted above the branches. *Renilla* luciferase probably evolved from haloalkane dehalogenase enzymes based on the tree topology.

Fig. **2** provides a posterior probability distribution of inferred amino acids across all sites of AncHLD-RLuc (SEQ ID NO: 1). The inferred amino acid at each position of the ancestral protein is the amino acid with the highest-weighted posterior probability at a given site.

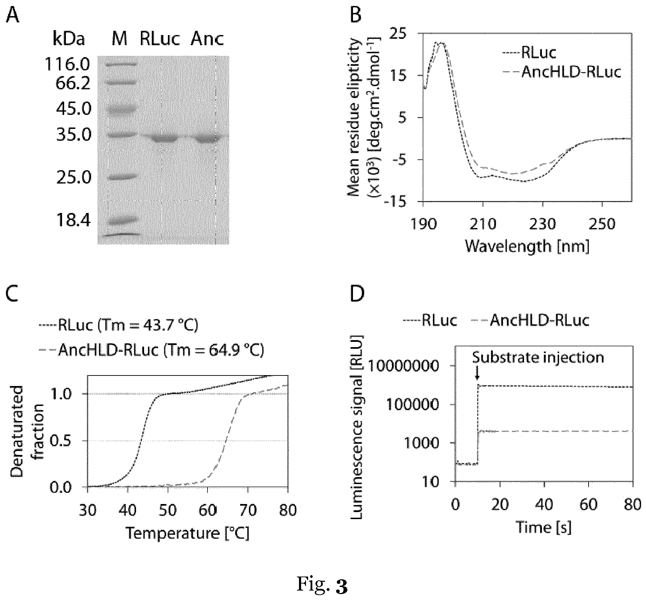

Fig. **3** summarizes the functional experimental characterization of the reconstructed ancestral protein AncHLD-RLuc (SEQ ID NO: 1). (A) SDS-PAGE gel of the purified AncHLD-RLuc confirms that the protein was successfully overproduced and isolated with the purity higher than 95 %. (B) Circular dichroism spectra showed that AncHLD-RLuc was correctly folded and retained the same overall fold as related haloalkane dehalogenases and the *Renilla* luciferase. (D) Thermostability measurement confirmed that the ancestral sequence reconstruction strategy yielded a hyperstabilized protein with an increased melting temperature, allowing to accept major structural modifications. (E) Bioluminescence signal measurement after the addition of the coelenterazine substrate proved that AncHLD-RLuc possessed bioluminescence Renilla-like activity.

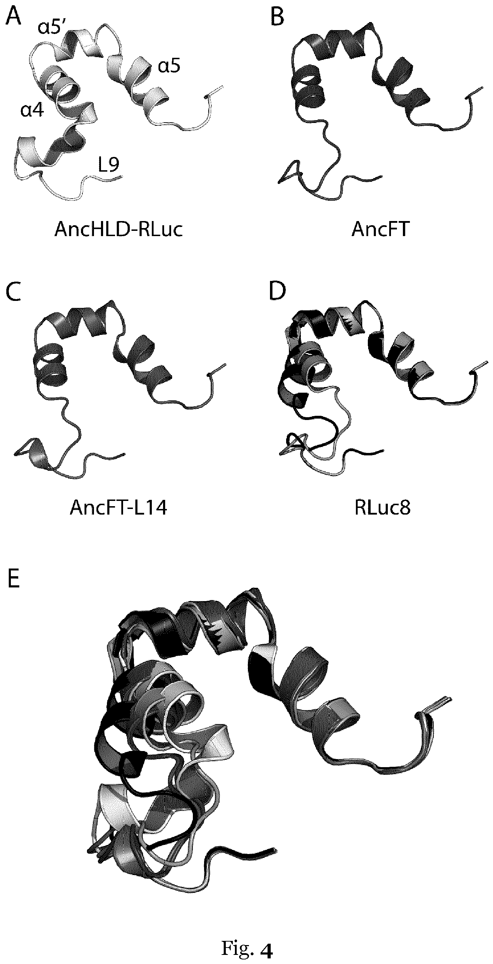

Fig. **4** shows the crystal structures of AncHLD-RLuc (A), AncFT (B), AncFT-L14 (C), RLuc8 (D), and all structures aligned for comparison (E). RLuc8 has two monomers in the asymmetric unit differing in the conformation of the $\alpha$4 helix. Chains A (black) and B (grey) are in the open and closed conformations, respectively. AncFT and AncFT-L14 have one monomer in the crystal lattice in which helix $\alpha$4 is more open than in AncHLD-RLuc, and even RLuc8 chain B.

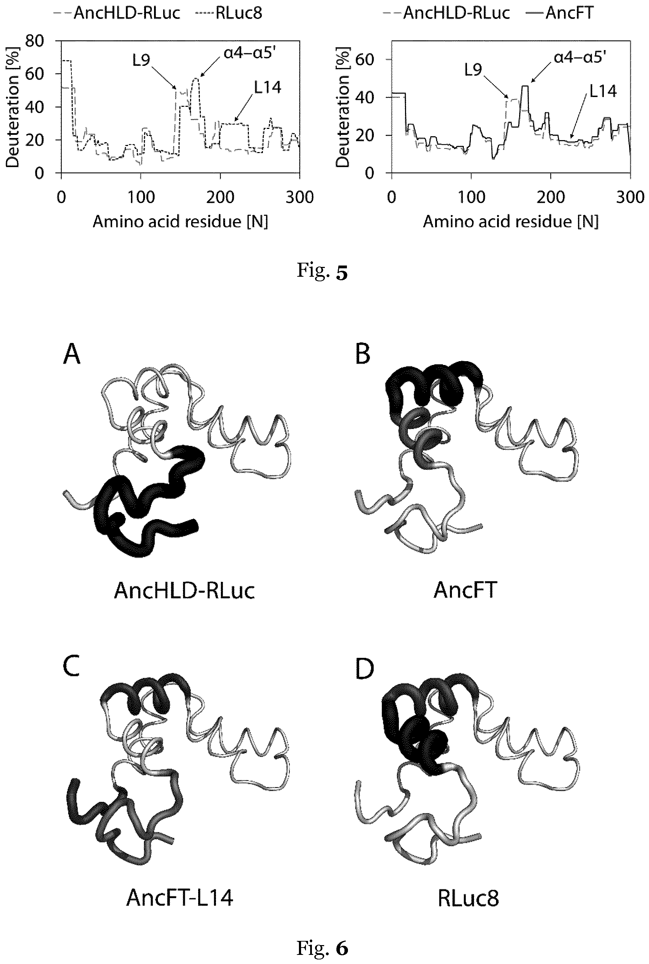

Fig. **5** displays dynamics and hydration of AncHLD-RLuc, AncFT, and RLuc8 based on HDX-MS assessments of peptide deuteration after 60 seconds. The experiment was carried out at room temperature and was quenched by the addition of 1 M HCl in 1 M glycine with pepsine. Freshly prepared sample of AncHLD-RLuc was always used as a template since the analysis of AncFT and RLuc8 was done in different time periods.

Fig. **6** illustrates conformational dynamics of AncHLD-RLuc (A), AncFT (B), AncFT-L14 (C), and RLuc8 (D) observed in molecular dynamics simulations. B-factors were calculated for backbone atoms, standardized, and averaged for the secondary structure elements. Color range from low (light grey) to high (black) B-factor is the same across all the proteins, while thickness ranges from low to high B-factor for each protein separately.

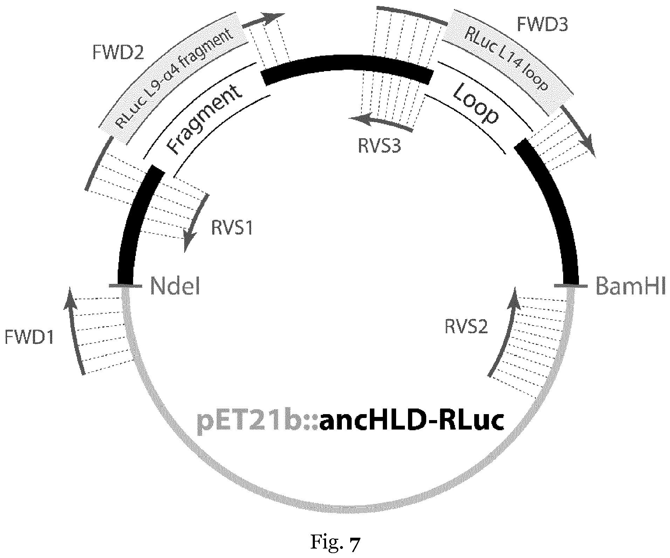

Fig. 7 illustrates schematic representation of hybridization of primers designed for fragment transplantation of the ancestral protein (SEQ ID NO: 1) to generate novel coelenterazine-utilizing luciferases by transplanting the L9-$\alpha$4 region and/or L14 region. Primer names correspond to those stated in Table II.

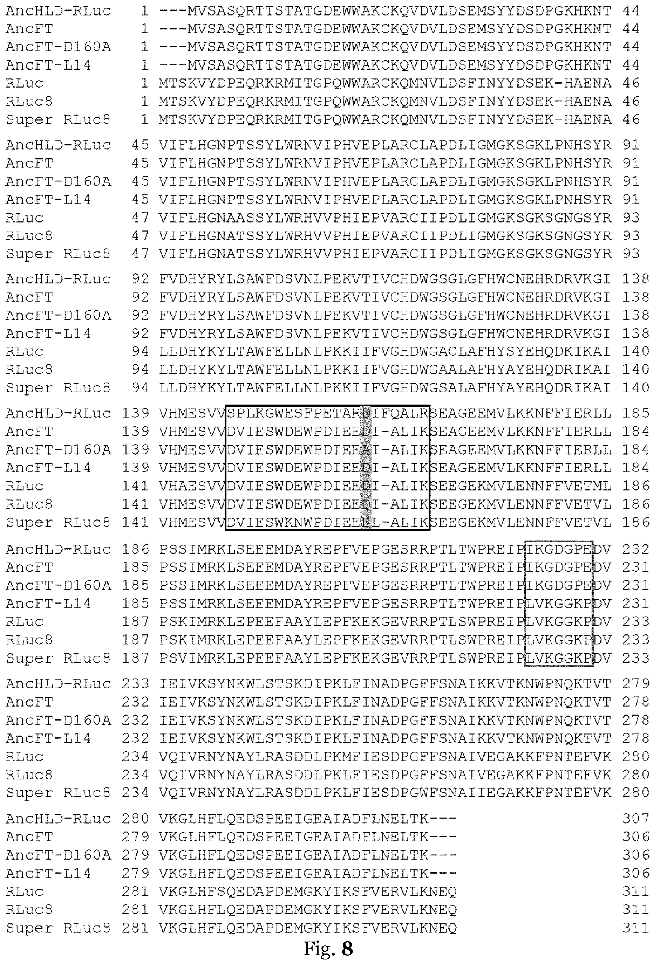

Fig. **8** provides sequence alignment of the ancestral protein, novel coelenterazine-utilizing luciferases (AncFT, AncFT-L14, and AncFT-D160A), and *Renilla reniformis* luciferase RLuc and the mutant variants RLuc8 and Super RLuc8. The regions selected for fragment transplantation are highlighted with the rectangles (black rectangle marking the L9 loop and the $\alpha$4 helix regions, grey rectangle marking the L14 loop) while the D160A mutation is highlighted with a grey background.

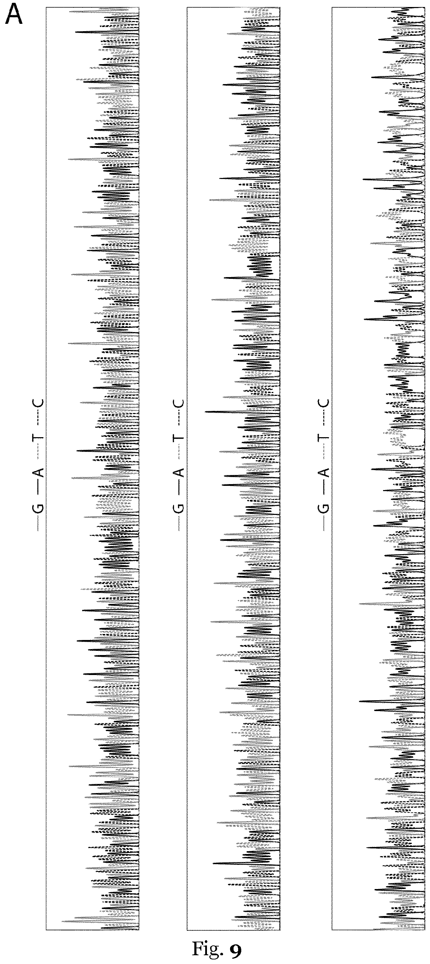

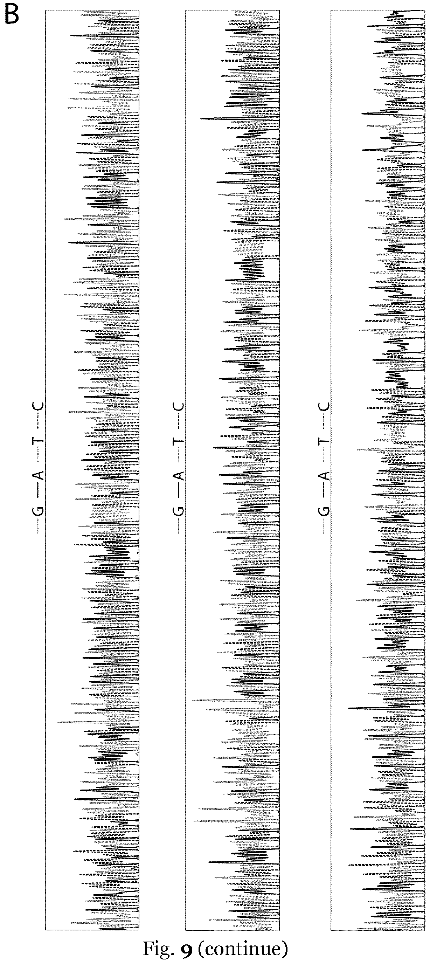

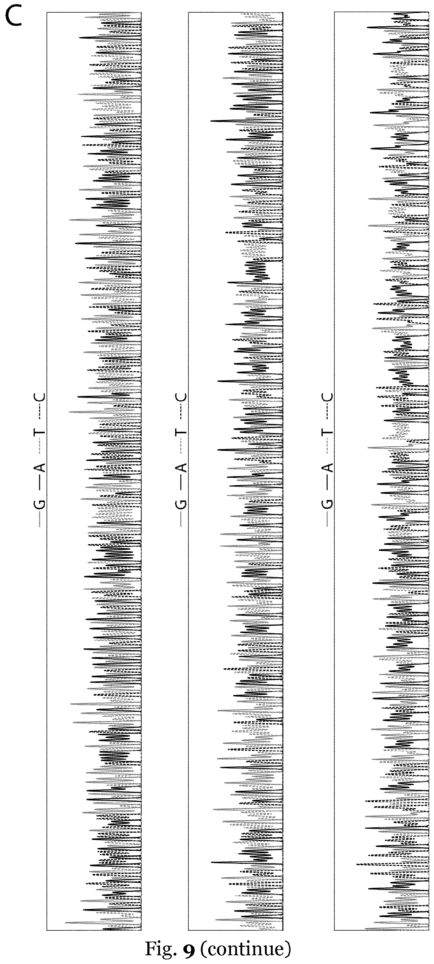

Fig. **9** shows sequencing chromatograms of the newly constructed DNA sequences encoding luciferases AncFT (A), AncFT-D160A (B), and AncFT-L14 (C). All the sequences have the corresponding length and the correct sequence of nucleotides, as designed by the fragment transplantation strategy.

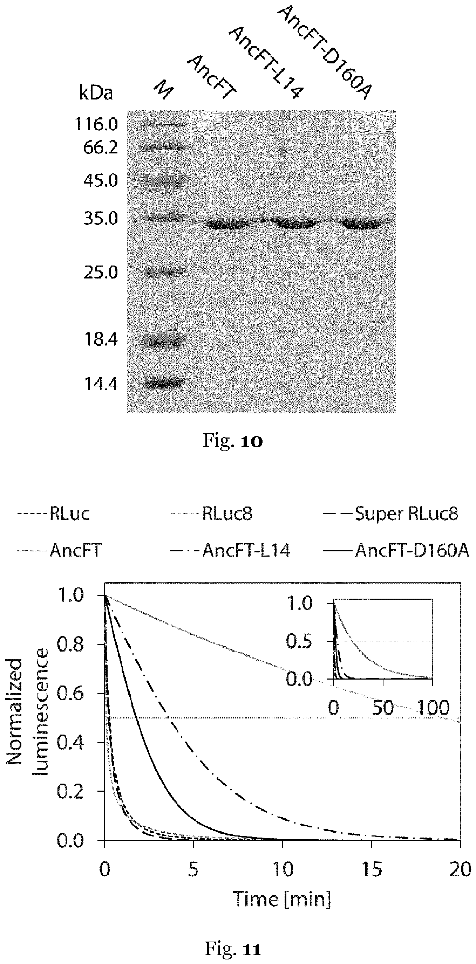

Fig. **10** displays stained SDS-PAGE gels of the newly constructed purified coelenterazine-utilizing luciferases AncFT, AncFT-L14, and AncFT-D160A. The marker lane is labelled with the letter M, molecular weights of standards are shown next to the corresponding bands. The purity of all proteins was higher than 95 %.

Fig. **11** presents the bioluminescence decay curves collected for coelenterazine-utilizing *Renilla-type* luciferase enzymes at the same concentrations of the coelenterazine substrate (2.2 $\mu$M) and an enzyme (50 nM). The figure clearly distinguishes between variants with the flash-type bioluminescence emission (fast decay after a strong initial flash) and the glow-type bioluminescence emission (stable signal with slow decay). Each experiment was measured in 3 replicates in 100 mM potassium phosphate buffer pH 7.5 at 37 °C. The smaller insert graph presents the same bioluminescence signals but with a longer timescale (X-axis) to show the decay of the glow-type luciferase variants.

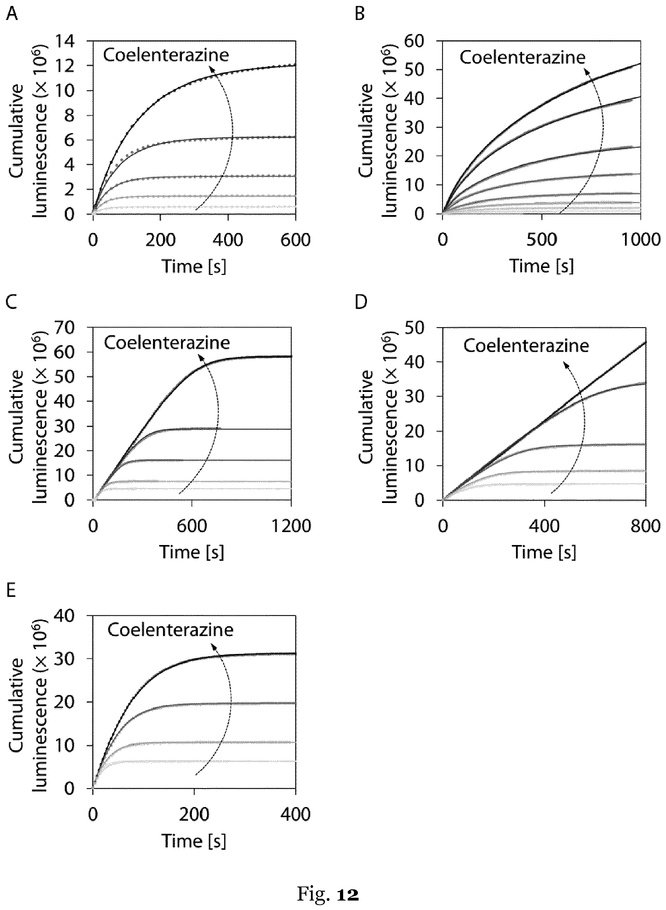

Fig. **12** summarizes full-conversion cumulative luminescence kinetic curves collected for coelenterazine-utilizing *Renilla-type* luciferase enzymes at various substrate concentrations, allowing assessment of steady-state kinetic parameters, including Michaelis constant $K_m$, turnover number $k_{cat}$, and enzyme-product complex dissociation constant $K_p$. The data were collected for RLuc8 (A), Super RLuc8 (B), AncFT (C), AncFT-L14 (D), and AncFT-D160A (E). Solid lines represent the best fit. Each experiment was measured in 3 replicates in 100 mM potassium phosphate buffer pH 7.5 at 37 °C.

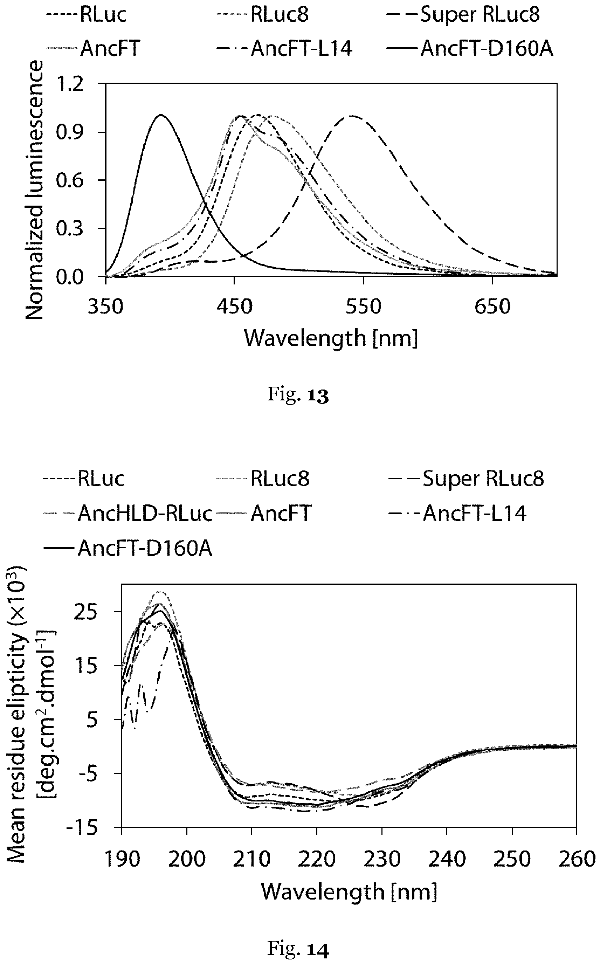

Fig. **13** compares the bioluminescence emission spectra of various coelenterazine-utilizing luciferases and emphasizes differences in the emission peak maxima positions. The spectra were collected in 100 mM potassium phosphate buffer pH 7.5 at ambient temperature.

Fig. **14** shows comparison of far-UV circular dichroism spectra of coelenterazine-utilizing luciferases. The spectra were collected in 5 repetitions and baseline-corrected for the blank composed of 100 mM potassium phosphate buffer pH 7.5.

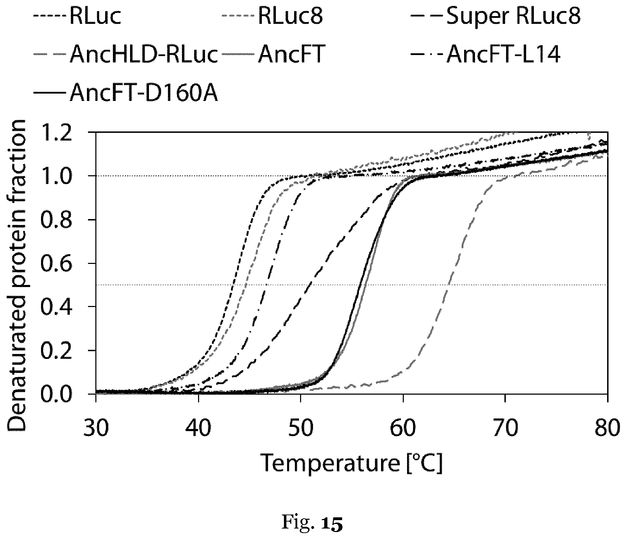

Fig. **15** provides melting curves of tryptophan fluorescence changes converted to fractions of denatured protein with increasing temperature. The temperature dependent curves were collected for coelenterazine-utilizing luciferase enzymes in 100 mM potassium phosphate buffer pH 7.5. Solid lines represent the best fit.

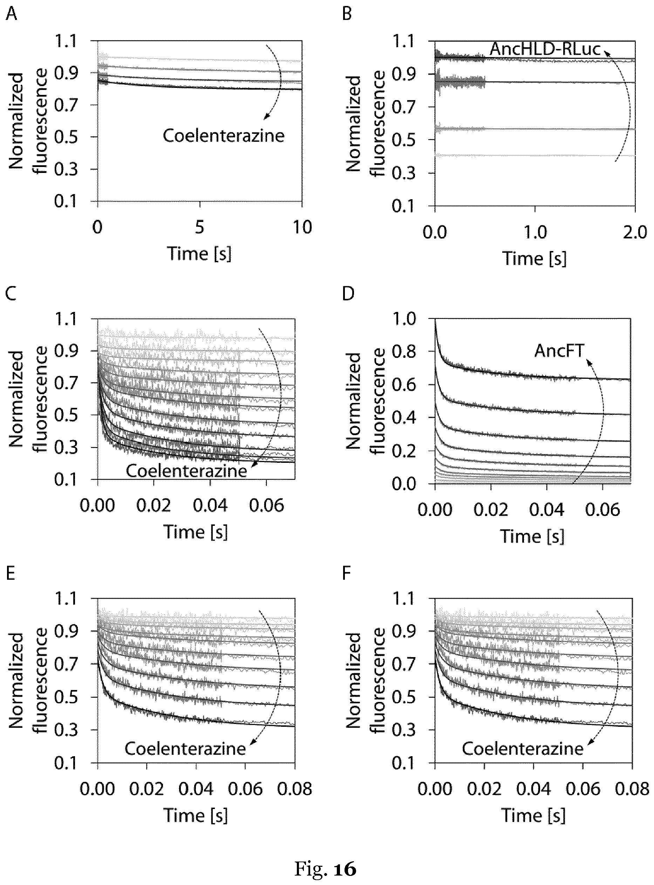

Fig. **16** presents pre-steady-state time course of the tryptophan fluorescence signal quenching upon binding of the coelenterazine substrate by coelenterazine-utilizing luciferase enzymes AncHLD-RLuc (A and B), AncFT (C and D), and RLuc8 (E and F). The data were collected at both variable substrate concentrations (A, C, and E) and variable enzyme concentrations (B, D, and F). The experiments were performed in 100 mM potassium phosphate buffer pH 7.5 at 15 °C. Each fluorescence trace was measured in 7 consecutive replicates and averaged. Solid lines represent the best fit.

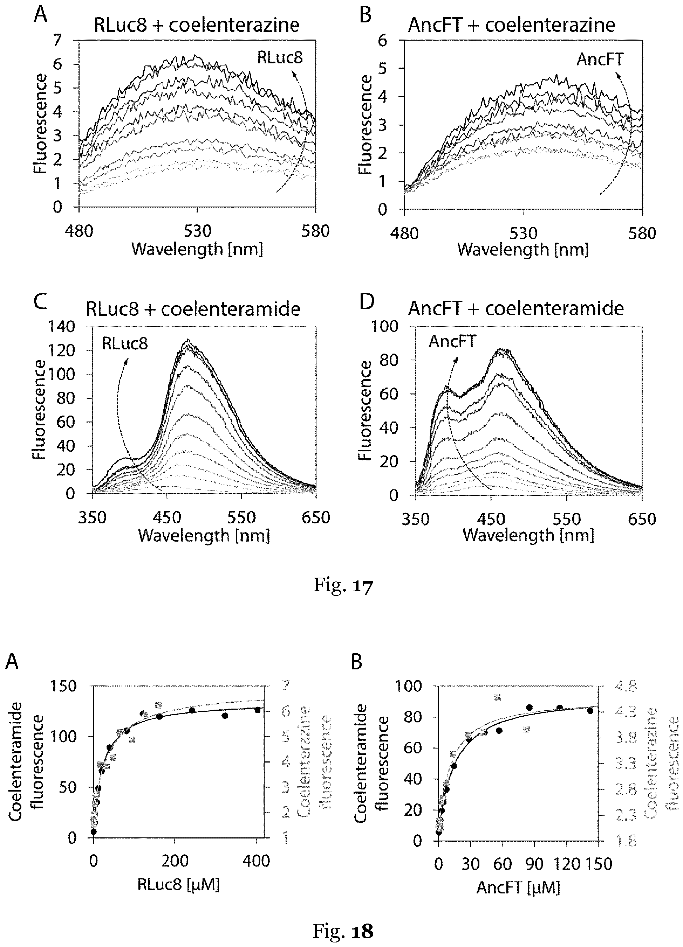

Fig. **17** demonstrates fluorescence emission spectra of coelenterazine (A and B) and coelenteramide (C and D) and their changes upon the ligand binding by coelenterazine-utilizing luciferases RLuc8 (A and C) and AncFT (B and D). Coelenterazine emission spectra were collected upon excitation at 420 nm while coelenteramide spectra upon excitation at 330 nm. The data were measured under anaerobic conditions in 100 mM potassium phosphate buffer pH 7.5 at 30 °C.

Fig. **18** displays concentration dependence of coelenterazine/coelenteramide fluorescence peak maxima with increasing concentration of RLuc8 (A) and AncFT (B) due to the binding by the enzymes. The data were collected under anaerobic conditions in 100 mM potassium phosphate buffer pH 7.5 at 30 °C. Solid lines represent the best fit according to the hyperbolic relationship.

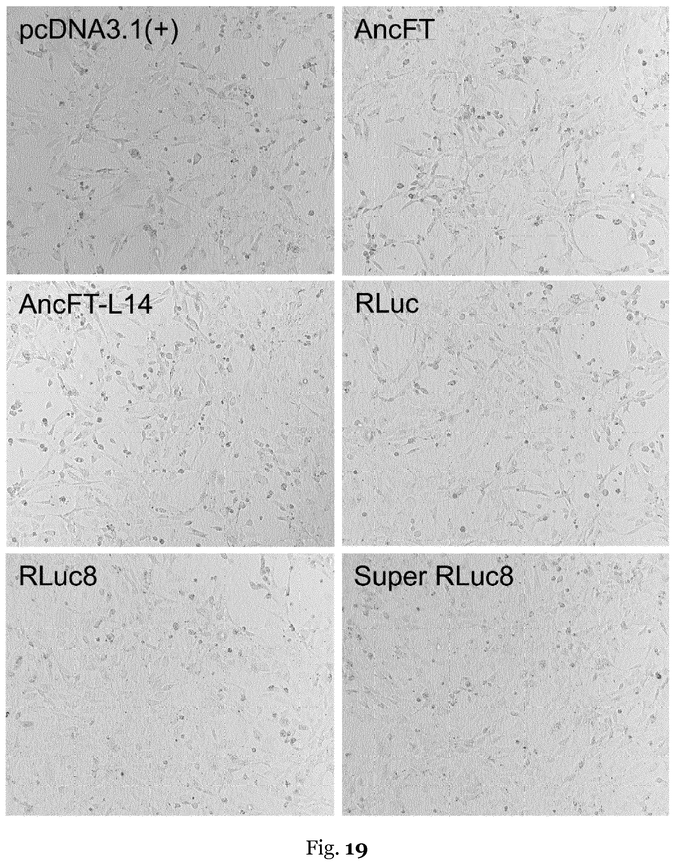

Fig. **19** provides micrographs of NIH/$_3$T$_3$ mouse fibroblast cells transfected with an empty pcDNA$_{3.1}$(+) plasmid, or the pcDNA$_{3.1}$(+) plasmid carrying a gene encoding the luciferase AncFT, AncFT-L14, RLuc, RLuc8, or Super RLuc8.

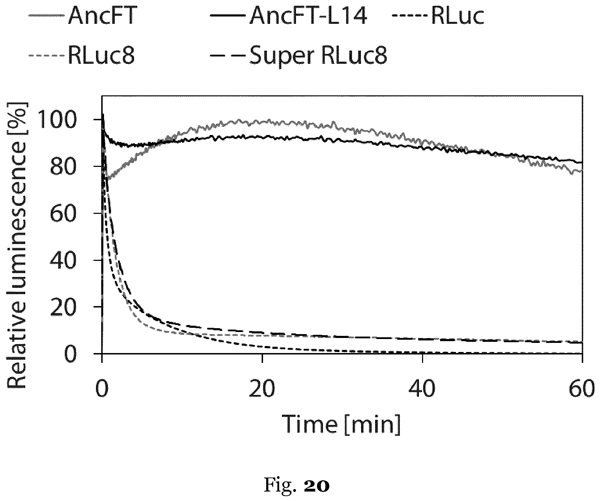

Fig. **20** compares bioluminescence signal stabilities of heterologously expressed luciferases AncFT, AncFT-L14, RLuc, RLuc8, and Super RLuc8 in the mammalian cells lysate. The data were measured using the commercial *Renilla* luciferase assay kit (Promega) with 20 µL of cell lysates and 100 µL of assay buffer.

Examples

**Example 1. Generation of a phylogenetic tree and reconstruction of the maximum likelihood sequence of an ancestral *Renilla*-type luciferase**

To reconstruct the ancestral sequence, a non-redundant set of related haloalkane dehalogenase and *Renilla* luciferase protein sequences was collected by PSI-BLAST v2.2.22+ (Altschul et al., (1997). Nucleic Acids Res. 25, 3389-3402.) searches against the NCBI nr database, version 21-9-2012 (NCBI Resource Coordinators, (2013). Nucleic Acids Res. 41, D8-D20.), then clustered with CLANS (Frickey & Lupas, (2004). Bioinformatics. 20, 3702-3704.) and CD-HIT v4.5.4 (Li & Godzik, (2006). Bioinformatics. 22, 1658-1659.). The dataset was divided into two sub-groups based on their evolutionary relatedness. For each sub-group, a separate multiple sequence alignment (MSA) was constructed by MUSCLE v3.51 (Edgar, (2004). BMC Bioinformatics. 5, 113.) and refined in BioEdit (Hall, (1999). Nucleic Acids Symp. Ser. 41, 95-98.) based on the manually prepared structure-guided MSA. The MSAs of both sub-groups were then aligned to each other using MUSCLE profile-profile alignment and manually refined.
The phylogenetic tree of the aligned protein sequences was calculated by PhyML v3.0 (Guindon et al., (2010). Syst. Biol. 59, 307-321.), based on the LG substitution matrix (Le & Gascuel, (2008). Mol. Biol. Evol. 25, 1307-1320.) combined with +1, +F and +G parameters selected by PROTTESTv2.4 (Abascal et al., (2005). Bioinformatics. 21, 2104-2105.) and using the best result from nearest-neighbor interchange (Guindon & Gascuel, (2003). Syst. Biol. 52, 696-704.) and subtree pruning and regrafting (Hordijk & Gascuel, (2005). Bioinformatics. 21, 4338-4347.) to optimize the tree topology starting from BIONJ (Gascuel, (1997). Mol. Biol. Evol. 14, 685-695.) or five random starting trees, respectively. Confidence levels of the output tree were estimated by bootstrapping the data 100 times. The tree was rooted according to the previously published haloalkane dehalogenase tree (Chovancova et al, (2007). Proteins. 67, 305-316.). For all ancestral nodes of each tree from the obtained distribution of trees, ancestral states at each site of the MSA were predicted by the maximum-likelihood method (Koshi & Goldstein, (1996). J. Mol. Evol. 42, 313-320.) using the Codeml module of PAML v4.4 (Yang, (2007). Mol. Biol. Evol. 24, 1586-1591.) and Lazarus software (Hanson-Smith, Kolaczkowski & Thornton, (2010). Mol. Biol. Evol. 27, 1988-1999.) based on the LG+F+G model. The ancestral state distributions at each site and each node were then obtained by weighting ancestral reconstructions from each tree by the associated posterior probability of a given tree. The most probable ancestor sequence of the selected node was then predicted by assigning to each position the ancestral state with the highest-weighted posterior probability. The ancestral gap characters were initially placed by Lazarus according to Fitch's parsimony (Fitch, (1971). Syst. Zool. 20, 406-416.) and then refined manually in BioEdit.
The generated phylogenetic tree of the aligned protein sequences is provided in Fig. **1** while the posterior probability distribution of inferred amino acids across all sites of AncHLD-RLuc (SEQ ID NO: 1) is shown in Fig. **2**. The phylogenetic tree clearly shows that haloalkane dehalogenases and the *Renilla*-luciferase RLuc share a high degree of sequence similarity and most probably, the two groups of enzymes are evolutionarily related. It is also very likely that the *Renilla*-luciferase evolved from haloalkane dehalogenases and thus, the reconstructed sequence of the ancestral protein AncHLD-RLuc represents the maximum likelihood sequence of a starting point of the divergent evolution process. The most probable sequence of this protein was inferred by assigning the ancestral state with the highest-weighted posterior probability to each site based on 93 sequences available for the homologous enzymes. The AncHLD-RLuc sequence has 56 % sequence identity with the *Renilla*-luciferase and 64 % sequence identity with the closest characterized modern-day haloalkane dehalogenase DspA (Fortova et al., (2013). Biochimie. 95, 2091-2096.). The sequence of the ancestral protein further contains the catalytic pentad Asp-His-Glu+Trp-Asn that is preserved in both the luciferase and the dehalogenase enzymes.

**Example 2. Experimental construction and functional characterization of a reconstructed protein sequence of an ancestral *Renilla*-type luciferase**

The gene encoding the reconstructed sequence of the ancestral protein AncHLD-RLuc (SEQ ID NO: 1) was synthetized artificially (GeneArt, Germany). The codon usage was automatically adapted to the codon bias of *Escherichia coli* genes by GeneArt's website service. For expression purposes, the gene was subcloned into the expression vector pET21b (Novagen, USA) between NdeI and BamHI restriction sites. The resulting vector pET21b::ancHLD-RLuc was inserted into chemocompetent *E. coli* BL21(DE3) cells by the heat shock method. The transformed cells were assayed on agar plates supplemented with ampicillin (100 $\mu$g/mL). Ampicillin-resistant colonies were used for further experiments.

Overexpression of AncHLD-RLuc (under the control of the T7lac promoter) was carried out in *E. coli* BL21(DE3) cells cultivated in Luria broth (LB) medium supplemented with ampicillin (100 $\mu$g/mL) at 37 °C. The cells were grown (200 rpm, 37 °C) until the culture reached $OD_{600} \approx 0.6$. Induction of expression was done at 20 °C by adding IPTG to the final concentration of 0.5 mM, and the culture was then incubated overnight (typically 12-16 h) at 20 °C and 115 rpm. Next day, the bacterial biomass was harvested by centrifugation (3,600×g, 10 min, 4 °C), re-suspended in a purification buffer (500 mM NaCl, 20 mM potassium phosphate buffer pH 7.5, 10 mM imidazole), and stored at -70 °C.

Prior to overproduced protein isolation and purification, the harvested cells were lysed by sonication using an UP200S ultrasonic processor (Hielscher, Germany) and centrifuged for 60 min at 4 °C and 21,000×g. The supernatant was then collected, filtered, and applied on a Ni-NTA Superflow Cartridge column (Qiagen, Germany) equilibrated with the purification buffer. The target enzyme was eluted by a linear gradient of the purification buffer supplemented with 300 mM imidazole. The eluted fractions of the target protein were pooled and dialyzed overnight against 100 mM potassium phosphate buffer pH 7.5. The dialyzed protein was subsequently loaded onto 16/60 Superdex 200 gel filtration column (GE Healthcare, UK) pre-equilibrated with the corresponding buffer.

The purified protein was concentrated with an Amicon Ultra centrifugal filter units (Millipore, USA), and protein concentrations were determined by the DS-11 Spectrophotometer (DeNovix, USA). Purity of the prepared luciferase proteins was checked by the SDS-PAGE method using Mini-PROTEAN Tetra (Bio-Rad, USA). The vertical gel electrophoresis was run under denaturing conditions (120 V, 80 minutes), and the resulting gels were stained by the InstantBlue solution (Sigma-Aldrich, USA) and then scanned using densitometer GS-800 Calibrated (Bio-Rad, USA).

Circular dichroism spectra, providing information about the secondary structure composition and correct folding of proteins, were recorded at 20 °C using a spectropolarimeter Chirascan (Applied Photophysics, United Kingdom). Data were collected from 185 to 260 nm at 100 nm/min with a 1 second response time and a 1 nm bandwidth in a 0.1 cm quartz cuvette. Each spectrum was measured in five repetitions and was subsequently corrected for the absorbance of the buffer. The circular dichroism data were expressed in terms of the mean residue ellipticity $\Theta_{MRE}$, calculated according to Equation (1) where $\Theta_{obs}$ is the observed ellipticity in degrees, $M_w$ is the protein molecular weight, n is the number of residues, 1 is the cell path length, c is the protein concentration (in mg/mL) and the factor of 100 originates from the conversion of the molecular weight to mg/dmol.

$$\Theta_{MRE} = \frac{\Theta_{obs} \cdot M_w \cdot 100}{n \cdot c \cdot l} \qquad (1)$$

Thermal unfolding was studied using NanoDSF Prometheus (NanoTemper, Germany) by monitoring tryptophan fluorescence over the temperature range of 20 to 90 °C, at a heating rate of 1 °C/min. The melting temperatures ($T_{onset}$ and $T_m$) were evaluated directly by ThermControl v2.0.2.

Luminescence activity was determined using the luminescence microplate reader FLUOstar OPTIMA (BMG Labtech, Germany) by automatic addition of 225 $\mu$L of a buffer solution of coelenterazine into a microplate well containing 25 $\mu$L of an enzyme solution. Both the coelenterazine substrate and the AncHLD-RLuc enzyme were dissolved in 100 mM potassium phosphate buffer pH 7.5 and their resulting concentrations in the reaction mixture were 7.9 $\mu$M coelenterazine, 1.5 nM RLuc, and 2.8 $\mu$M AncHLD-RLuc. Bioluminescence activity traces were collected at 37 °C over the first 72.5 seconds immediately after injection of the substrate into the enzyme solution. The activity of each enzyme sample was measured in at least three independent experiments. The collected luminescence curves were integrated to obtain a linear increase of cumulative luminescence over time, while the slope gave the final value of activity, represented in RLU.s$^{-1}$. For each enzyme, the calculated activity was related to one milligram of the enzyme to get the final value of specific activity in RLU.s$^{-1}$.mg$^{-1}$.

The overview of the biochemical characterization of the generated ancestral protein AncHLD-RLuc is summarized in Fig. **3.** The stained SDS-PAGE gel analysis showed that the ancestral protein was successfully prepared and isolated with the purity higher than 95 %. The protein was overproduced with an average yield of 250 mg of soluble purified protein per one liter of cell culture. The follow-up circular dichroism characterization pointed out that the protein kept the same overall $\alpha/\beta$-hydrolase fold as the spectrum was very similar to that of the *Renilla*-luciferase RLuc. The thermal denaturation experiment showed that AncHLD-RLuc had a greater thermal stability than RLuc, with 21.2 °C higher apparent melting temperature. Most importantly, the ancestral enzyme also exhibited a clearly detectable bioluminescence activity, as proved by the luminescence activity measurements. However, the activity was much lower (>100,000-fold) compared to the modern luciferase enzyme. Collectively, these findings suggested that AncHLD-RLuc was an interesting system with a good potential for further laboratory evolvability as it was hyperstabilized and thus could accept even more drastic structural backbone modifications and at the same time, it exhibited only a very low luciferase bioluminescence activity, leaving a lot of free space for further improvement by the tools of protein engineering.

**Example 3. Identification of important regions for bioluminescence activity by crystallographic analysis**

Purified AncHLD-RLuc enzyme, concentrated to 10-15 mg/mL and dissolved in 50 mM Tris-HCl buffer pH 7.5 was used for crystallization screening. Crystals suitable for X-ray diffraction analysis were obtained from a JCSG-plus screen (Molecular Dimensions, UK) at 292 K using the sitting-drop vapor-diffusion method with a protein:precipitant ratio of 1:1. The crystallization solution consisted of 0.1 M HEPES pH 7.0 and 10 % (w/v) PEG 6000. Single crystals of AncHLD-RLuc with average dimensions of $70.6 \times 39.7 \times 8.8$ $\mu$m were cryoprotected by soaking them in a drop containing the crystallization solution supplemented with 25 % (v/v) 2-methyl-2,4-pentanediol (MPD) for 5 seconds. The crystals were then mounted in a SPINE cryoloops (MiTeGen, USA) and flash-cooled in liquid-nitrogen. Diffraction data were collected at the Diamond Light Source beamline I02 to 1.39 Å resolution at 0.9795 Å wavelength using a Pilatus 6M-F detector. In total, 1,200 images were collected at 100 K with an oscillation range of 0.15 ° per image. The data were processed with Mosflm v7.1.0 (Battye et al., (2011). Acta Crystallogr. D Biol. Crystallogr. 67, 271-281.) using the first 900 images for indexing and integration, and Aimless vo.2.17 (Evans & Murshudov, (2013). Acta Crystallogr. D Biol. Crystallogr. 69, 1204-1214.) for scaling and merging.

The structure of the protein was solved by molecular replacement using Phaser software (McCoy, (2007). J. Appl. Crystallogr. 40, 658-674.) implemented in the CCP4 package (Winn, (2011). Acta Crystallogr. D Biol. Crystallogr. 67, 235-242.) and using the structure of RLuc8 from *Renilla reniformis* (PDB ID 2PSF) as a search model. Manual model building was performed in Coot vo.8.1 (Emsley & Cowtan, (2004). Acta Crystallogr. D Biol. Crystallogr. 60, 2126-2132.) using $2F_o$-$F_c$ and $F_o$-$F_c$ maps, followed by several cycles of automated refinement with REFMAC v5.8 (Murshudov, Vagin & Dodson, (1997). Acta Crystallogr. D Biol. Crystallogr. 53, 240-255.) from the CCP4 package and phenix.refine from the Phenix package (Adams, (2010). Acta Crystallogr. D Biol. Crystallogr. 66, 213-221.) using the anisotropic B-factor model. The final refinement step and model optimization was performed using PDB_REDO (Joosten, (2014). IUCrJ. 1, 213-220.). Figures showing structural representations were prepared using The PyMOL Molecular Graphics System, version 1.5 (Schrödinger, USA). Atomic coordinates and experimental structure factors have been deposited in the Protein Data Bank under the PDB code 6G75.

AncHLD-RLuc was successfully crystallized and its tertiary structure was solved by X-ray crystallography to 1.39 Å resolution, with two copies of the enzyme in the asymmetric unit. The refinement statistics are provided in Table I. The crystal belonged to the primitive monoclinic space group P12$_1$1 with the unit-cell parameters a = 44.549, b = 60.51, c = 123.63 Å, $\alpha = \gamma = 90$ °, $\beta = 90.9$ °. The Matthews coefficient ($V_M$) (Kantardjieff & Rupp, (2003). Protein Sci. Publ. Protein Soc. 12, 1865-1871.) of 2.32 Å$^3$.Da$^{-1}$ showed that the crystal contained two molecules per asymmetric unit with estimated solvent content of 46.93 %. The overall structure of AncHLD-RLuc was comprised of an $\alpha/\beta$ hydrolase core domain and a helical cap domain. The hydrophobic cavity is located between the core and the cap domain. The cavity is connected to the protein surface by two access tunnels with the main access tunnel lined by elements including the $\alpha$4 helix of the cap domain.

When compared to RLuc8, the $\alpha$4 helix region together with the adjacent L9 loop exhibited the largest differences in their spatial arrangements, as shown in Fig. **4**. The asymmetric unit of the crystal lattice of RLuc8 contains two monomers (chains A and B). The chain B is similar to AncHLD-RLuc, while chain A is in an open conformation with the $\alpha$4 helix pointing away and making the active site much larger. As a consequence, the size of the active site cavity, and the width of the tunnel mouth and active site accessibility can be significantly affected by the conformational flexibility. Such finding indicated that the flexible L9-$\alpha$4 region was very important for active site accessibility and effective binding of the bulky coelenterazine substrate.

TABLE I

| Protein | AncHLD- RLuc |
|---|---|
| Space group | *P*12$_1$1 |
| Cell parameters | |
| *a, b, c* [Å] | 44.55, 60.51, 123.63 |
| $\alpha, \beta, \gamma$ [°] | 90, 90.9, 90 |

(continued)

| Protein | AncHLD- RLuc |
|---|---|
| Number of molecules in AU | 2 |
| Wavelength [Å] | 0.9795 |
| Resolution range [Å] | 21.48-1.39 (1.44-1-39) |
| Observed reflections | 1502788 (148042) |
| Number of unique reflections | 124697 (11940) |
| Completeness [%] | 92.62 (91.12) |
| Multiplicity | 12.1 |
| $1/\sigma$ [I] | 20.6 (1.65) |
| $R_{merge}$ | 0.6 (1.8) |
| $CC_{1/2}$ | 0.8 (0.324) |
| Refinement statistics | |
| Resolution [Å] | 1.39 |
| No. of reflections in working set $R_{work}$ | 122118 (11933) 0.15 |
| $R_{free}$ | 0.19 |
| RMSD bond length [Å] | 0.005 |
| RMSD angle [°] | 0.821 |
| Contents of asymmetric unit | |
| No. of atoms in AU | 5338 |
| No. of protein atoms in AU | 4867 |
| No. of water molecules in AU | 465 |
| No. of ligand atoms | 36 |
| Mean B values | |
| Protein [Å$^2$] | 16.99 |
| Ligands $O_2$/MPD/Tris [Å$^2$] | 46.68 |
| Waters [Å$^2$] | 32.87 |
| PDB code | 6G75 |

## Example 4. Identification of important regions for bioluminescence activity by hydrogen-deuterium exchange mass spectrometry (HDX-MS)

HDX-MS experiments were carried out at room temperature in 100 mM potassium phosphate buffer pH 7.5. Enzyme samples were diluted with 100 mM phosphate buffer in $H_2O$ (pH 7.5), to prepare undeuterated controls and for peptide mapping, or with 100 mM phosphate buffer in $D_2O$ (pD 7.1) to prepare deuterated samples. The final concentrations of the enzyme samples used for analysis was 2 $\mu$M. The deuteration process, initiated by dilution samples in deuterated buffer, was quenched after 10 s, 60 s, or 300 s by adding 1 M HCl in 1 M glycine with pepsin. Each sample was directly injected into the LC-system UltiMate 3000 RSLCnano (Thermo Fisher Scientific, USA) with an immobilized nepenthesin enzymatic column (Affipro, Czech Republic; 15 $\mu$L bed volume, flow rate 20 $\mu$L/min, 2 % acetonitrile/0.05 % trifluoroacetic acid). Peptides were trapped and desalted on-line on a peptide microtrap (Michrom Bioresources, USA) for 3 minutes at the flow rate of 20 $\mu$L/min. Next, the peptides were eluted onto an analytical column (Jupiter C18, 1.0 x 50 mm, 5 $\mu$m, 300 Å, Phenomenex, USA) and separated by a linear gradient elution starting with 10 % of the buffer A in the buffer B and rising to 40% of the buffer A over 2 minutes. This was followed by 31 minutes of isocratic elution at 40 % of the buffer A. Buffers A and B consisted of 0.1 % formic acid in water and 80 % acetonitrile/0.08 % formic acid, respectively.

Mass spectrometric analysis was carried out using an Orbitrap Elite mass spectrometer (Thermo Fisher Scientific, USA) with ESI ionization connected on-line to a robotic system based on the HTS-XT platform (CTC Analytics, Switzerland). The instrument was operated in a data-dependent mode for peptide mapping (HPLC-MS/MS). Each MS scan was followed by MS/MS scans of the three most intensive ions from both CID and HCD fragmentation spectra. Tandem mass spectra were searched using SequestHT against the cRap protein database containing the sequences of AncHLD-RLuc and RLuc8 with the following search settings: mass tolerance for precursor ions of 10 ppm, mass tolerance for fragment ions of 0.6 Da, no enzyme specificity, two maximum missed cleavage sites, and no-fixed or variable modifications. The false discovery rate at the peptide identification level was set to 1 %. Sequence coverage was analyzed with Proteome Discoverer version 1.4 (Thermo Fisher Scientific, USA) and graphically visualized with the MS Tools application (Kavan & Man, (2011). Hydrog. Exch. Mass Spectrom. 302, 53-58.).

Analysis of deuterated samples was done in HPLC-LC-MS mode with ion detection in the orbital ion trap. The raw MS files together with the list of peptides (peptide pool) identified with high confidence characterized by requested parameters (retention time, XCorr, and charge) were processed using HDExaminer version 2.2 (Sierra Analytics, USA). The software analyzed the behavior of the proteins and peptides and generated uptake data that were mapped to the protein's amino acid sequence using the following procedure: (i) each residue was assigned the uptake data from any peptide solved with high confidence; (ii) medium confidence peptides were also accepted for positions without previously assigned data; (iii) low confidence peptides were rejected. The final uptake value (expressed as % of deuteration) assigned to each amino acid corresponded to the average of all assigned values for its position.

The HDX-MS profiles of the analyzed proteins are provided in Fig. **5.** The results of RLuc8 showed that the L14 loop together with the $\alpha$4-$\alpha$5' fragment exhibited greater solvation although the deuteration of its L9 loop was less extensive than in AncHLD-RLuc. Backbone amide hydrogen-deuterium exchange is particularly sensitive to hydrogen bonding and therefore reflects hydrogen bonding strength, conformational dynamics, and the solvent accessibility of protein structures. Thus, the HDX-MS results clearly indicate that high deuteration of the hot spot regions comprising the $\alpha$4 helix, L9 loop, and L14 loop is linked to enzyme flexibility and efficient catalysis. The regions encompassing the L9-$\alpha$4 fragment and L14 loop are important structural elements that line the main access tunnels connecting the buried active site to the surrounding solvent. Their motions are concerted and significantly affect the active site's volume while fully preserving the geometry of the catalytic residues.

### Example 5. Identification of important regions for bioluminescence activity by molecular dynamics (MD) simulations

MD simulations were carried out on the crystal structures of RLuc8 (PDB 2PSF) and AncHLD-RLuc (PDB 6G75). Hydrogen atoms were added to the aforementioned structures using the H++ web server (Gordon et al., (2005). Nucleic Acids Res. 33, W368-W371.), at pH 7.5. Water molecules from the crystal structures, which did not overlap with the protonated structures, were retained. The systems were solvated using the solvate module of High Throughput Molecular Dynamics (HTMD) in a cubical water box of TIP3P water molecules so that all atoms were at least 10 Å from the surface of the box (Doerr et al., (2016). J. Chem. Theory Comput. 12, 1845-1852.). Cl$^-$ and Na$^+$ ions were added to neutralize the protein charge and get a final concentration of 100 mM. The systems were equilibrated using the Equilibration_v$_2$ module of HTMD. The system was first minimized using the conjugate-gradient method for 500 steps, after which the system was heated and minimized as follows (i) 500 steps (2ps) of NVT heating, with the Berendsen barostat, to 310 K, with constraints on all heavy atoms of the protein; (ii) 2.5 ns of NPT equilibration, with the Langevin thermostat, with 1 kcal.mol$^{-1}$.Å$^{-2}$ constraints on all the heavy atoms of the protein; and (iii) 2.5 ns of NPT equilibration, with the Langevin thermostat, without constraints. During the equilibration simulations, holonomic constraints were applied to all hydrogen-heavy atom bond terms and the mass of hydrogen atoms was scaled by a factor of 4, enabling the use of a 4 fs timestep (Harvey, Giupponi & Fabritiis, (2009). J. Chem. Theory Comput. 5, 1632-1639.). This approach transfers some mass from the heavy atom to the hydrogen to slow the fastest vibrations in the hydrogen atom, which is acceptable because the thermodynamic properties of biological systems are insensitive to the distribution of atomic masses (Hopkins et al., (2015). J. Chem. Theory Comput. 11, 1864-1874.).

All the simulations employed periodic boundary conditions, the particle mesh Ewald method was used to treat interactions beyond a 9 Å cut-off, electrostatic interactions were suppressed > 4 bond terms away from each other, and the smoothing and switching of van der Waals and electrostatic interactions were cut-off at 7.5 Å (Harvey & De Fabritiis, (2009). J. Chem. Theory Comput. 5, 2371-2377.). HTMD was used to perform adaptive sampling of the RMSD of the C$\alpha$ atoms of residues 10 to 290. The 20 ns production runs were started with the files resulting from the equilibration using the settings from the final equilibration step. The trajectories were saved every 100 ps. The adaptive epochs were updated every 6 hours to run with a minimum of 5 simulations and a maximum of 10 simulations for the total maximum of 110 epochs using TICA in 1 dimension (Naritomi & Fuchigami, (2011). J. Chem. Phys. 134, 065101.). Adaptive sampling here refers to the MD method that performs simulations in small sequential batches of 10 simulations. Before the next batch of simulations is submitted, the first batch is analyzed considering a certain parameter (RMSD in this study) and the less sampled conformational regions are used to start a new batch. This way the method ensures that the sampled regions are maximized. This sampling analysis is done using Markov state models making a list of metastable states distributed through the conformational space.

B-factors were calculated from all snapshots obtained from the MD simulations for the backbone atoms of each enzyme. The Metric Fluctuation tool from the HTMD package was used to calculate the RMSF, from which B-factors were calculated using Equation (2). The B-factors obtained from the MD simulations were standardized for each enzyme to enable comparisons between enzymes. This was done using Equation (3). Here, Z is the standardized value, X is the original B-factor value, $\mu$ is the average of the B-factors, and $\delta$ is the sample standard deviation. The simulations were made into a simulation list using HTMD, water was filtered out, and crashed simulations with lengths below 20 ns were omitted. The total simulation time required for the calculations was in excess of 4 $\mu$s for each enzyme. The dynamics of the enzymes were evaluated based on the RMSD of the C$\alpha$ atoms of residues 10 to 290 to avoid problems due to the misleadingly high RMSD values of the dynamically free residues at each end of the protein. The data were clustered into 200 clusters using the MiniBatchKmeans algorithm. The implied timescale plot (based on a Markov state model with various lag times) was constructed to select a lag time for Markov model construction, using the RMSD of the full protein as a metric. Because the timescales mostly stabilized after 15 ns lag time, this value was used in the models to construct the 2 Markov states (open and closed). The final data were calculated from 1,000 bootstrapping runs using 50 % of the data.

$$B = \left[\frac{8 * \pi^2}{3}\right] * RMSF^2 \qquad (2)$$

$$Z = \frac{X - \mu}{\delta} \qquad (3)$$

The results of the MD simulations analysis are depicted in Fig. **6.** In order to complement HDX-MS experiments, which measure the exchange of backbone amide hydrogens with deuterium, backbone B-factors from MD simulations were used to characterize the dynamics of the system. The highest overall B-factor in the cap domain of RLuc8 occurs in the $\alpha$4 helix, followed by the $\alpha$5' helix and the L9 loop. The L14 loop has B-factor values similar to those of the L9 loop, but the rest of the cap domain has below-average B-factor values. The dynamic profile of the AncHLD-RLuc template differs significantly - unlike in RLuc8, the most pronounced dynamics occur in the L9 loop but no significant dynamics could have been observed for the $\alpha$4 helix and the L14 loop region. Consequently, the poor dynamics of AncHLD-RLuc prevented this enzyme from effective substrate binding and pronounced conformational flexibility. By this finding, it was further confirmed that the regions of the $\alpha$4 helix, L9 loop, and L14 loop were important elements for bioluminescence activity.

**Example 6. Generation of improved coelenterazine-utilizing luciferases by fragment transplantation of important regions**

In order to confirm importance of the identified flexible regions (helix $\alpha$4, loop L9, and loop L14), the corresponding fragment sequences were transplanted from RLuc to the hyperstabilized ancestral protein AncHLD-RLuc. The sequences of the ancestral protein (SEQ ID NO: 1) and RLuc were aligned using Jalview (Waterhouse et al., (2009). Bioinformatics. 25, 1189-1191.) and T-coffee (Notredame, Higgins & Heringa, (2000). J. Mol. Biol. 302, 205-217.) with default settings. For fragment transplantation, construct pET21b::ancHLD-RLuc (NdeI/BamHI) was chosen as a template for mutagenesis. Mutagenic primers (Sigma-Aldrich, USA), shown in Table II, were designed manually for separate PCR runs, as schematically presented in Fig. 7. For AncFT generation, only the primers FWD1, FWD2, RVS1, and RVS2 were used. For AncFT-L14 generation, the newly created construct pET21b::ancFT (NdeI/BamHI) was used as a template. In the next step, a standard fusion PCR protocol was used to fuse DNA fragments from the previous PCR runs. The resulting fused DNA fragments were cloned into pET21b vectors (https://www.addgene.org/vector-database/2550/). The error-free status of the clones was confirmed by sequencing (Eurofins Genomics, Germany).

TABLE II

| Primer | Length (nucleotides) | Sequence 5'- to 3'- |
|---|---|---|
| FWD1 | 20 | TAATACGACTCACTATAGGG |
| FWD2 | 114 | GGTATTGTTCACATGGAAAGCGTTGTGGATGTTATTGAAAGCTG GGATGAATGGCCTGATATCGAAGAAGATATTGCCCTGATTAAAA GCGAAGCCGGTGAAGAAATGGTTCTG |
| FWD3 | 67 | GACCTGGCCTCGTGAAATTCCGCTGGTTAAAGGTGGTAAACCGG ATGTGATTGAAATTGTGAAAAGC |

(continued)

| Primer | Length (nucleotides) | Sequence 5'- to 3'- |
|---|---|---|
| RVS1 | 24 | AACGCTTTCCATGTGAACAATACC |
| RVS2 | 19 | GCTAGTTATTGCTCAGCGG |
| RVS3 | 22 | CGGAATTTCACGAGGCCAGGTC |

In order to further generate single-point mutants of the created sequences, mutagenic primers with the desired mutation were used. The variant AncFT-D160A was prepared using pET21b::ancFT (NdeI/BamHI) as a template for mutagenesis while the template for the variant AncFT-L14-D160A generation was pET21b::ancFT-L14 (NdeI/BamHI). The mutagenic primers were designed manually and their sequences are available in Table III. The primers were ordered from Sigma-Aldrich (USA) and were subsequently used for the MegaPrimer generation by the first round of PCR amplification. The isolated MegaPrimer (this time as a PCR primer) was applied to the second round of PCR amplification with the same plasmid sequence as a template to yield the final desired sequence carrying the single-point D160A mutation. The error-free status of the clones was confirmed by sequencing (Eurofins Genomics, Germany).

TABLE III

| Primer | Length (nucleotides) | Sequence 5'-to 3'- |
|---|---|---|
| FWD | 45 | GAATGGCCTGATATCGAAGAAGCCATTGCCCTGATTAAAAGCGAA |
| RVS | 19 | pET Rvs (GCTAGTTATTGCTCAGCGG) |

The vectors carrying the genes of the modified luciferases were inserted into chemocompetent *E. coli* BL21(DE3) cells by the heat shock method. The transformed cells were assayed on agar plates supplemented with ampicillin (100 μg/mL). Ampicillin-resistant colonies were used for further experiments.

Overexpression of luciferase variants was carried out in *E. coli* BL21(DE3) cells cultivated in LB medium supplemented with ampicillin (100 μg/mL) at 37 °C. The cells were grown (200 rpm, 37 °C) until the culture reached $OD_{600} \approx 0.5$. Induction of expression was done at 22 °C by adding 0.5 mL of 1 M IPTG, and the culture was then incubated overnight (typically 12-16 h) at 22 °C and 115 rpm. Next day, the bacterial biomass was harvested by centrifugation (3,600×g, 10 min, 4 °C), re-suspended in a purification buffer (500 mM NaCl, 20 mM potassium phosphate buffer pH 7.5, 10 mM imidazole), and stored at -70 °C.

Prior to overproduced protein isolation and purification, the harvested cells were lysed by sonication (50 % amplitude, 3×2 min, 5 s pulse/5 s pause) using a sonicator Sonic Dismembrator Model 705 (Fisher Scientific, USA). The sonicated lysate was clarified by centrifugation (21,000×g, 60 min, 4 °C). The supernatant was then collected, filtered, and applied on Ni-NTA Superflow Cartridge column (Qiagen, Germany) equilibrated with the purification buffer. The target enzyme was eluted by a linear gradient of the purification buffer supplemented with 300 mM imidazole. The eluted fractions of the target protein were pooled and dialyzed overnight against 100 mM potassium phosphate buffer pH 7.5. The dialyzed protein was subsequently loaded onto 16/60 Superdex 200 gel filtration column (GE Healthcare, UK) pre-equilibrated with the corresponding buffer.

The purified protein was concentrated with an Amicon Ultra centrifugal filter units (Millipore, USA), and protein concentrations were determined by the DS-11 Spectrophotometer (DeNovix, USA). Purity of the prepared luciferase proteins was checked by the SDS-PAGE method using Mini-PROTEAN Tetra (Bio-Rad, USA). The vertical gel electrophoresis was run under denaturing conditions (120 V, 80 minutes), and the resulting gels were stained by the InstantBlue solution (Sigma-Aldrich, USA) and then scanned using densitometer GS-800 Calibrated (Bio-Rad, USA).

The sequence alignment of AncHLD-RLuc, AncFT, AncFT-D160A, AncFT-L14, RLuc, RLuc8, and Super RLuc8 is presented in Fig. **8.** The alignment shows that sequences of the transplanted regions differ significantly including an insertion/deletion, indicating possible importance of this region and its backbone geometry for the luciferase activity. Sequencing chromatograms, shown in Fig. **9,** confirmed correct length and nucleotide sequence of the generated DNA molecules with fragment-transplanted regions. Overexpression of these sequences and the subsequent purification of the overproduced proteins resulted in isolation of luciferases with the purity higher than 95 %, as determined by SDS-PAGE and shown in Fig. **10.** The overall yield of all the newly generated coelenterazine-utilizing luciferases was very high and even exceeded the capacity of the Ni-NTA Superflow Cartridge column (200-250 mg) when protein extracted from 1 L of bacterial culture (ca 3.6 g wet cell weight) was loaded.

**Example 7. Analysis of bioluminescence decay kinetics and determination of stability and half-life of the emission signal**

Solid coelenterazine was dissolved in ice-cold ethanol and stored under nitrogen atmosphere in dark glass vials at -20 °C. Before each measurement, concentration and maintained quality of the ethanol stock solutions was verified spectrophotometrically. A buffer solution of coelenterazine was prepared by mixing an appropriate volume of the ethanol stock solution with 100 mM phosphate buffer pH 7.5 immediately before the measurement. Tested luciferase enzyme samples were dissolved in the same 100 mM phosphate buffer pH 7.5. The enzymatic reaction was initiated inside the microplate reader FLUOstar OPTIMA (BMG Labtech, Germany) by automatic addition of 225 μL buffer solution of coelenterazine into a microplate well containing 25 μL of enzyme solution. The resulting bioluminescence activity traces were collected at 37 °C until the luminescence intensity decreased to less than 0.5 % of its maximal measured value. Each luminescence trace was measured in 3 repetitions. The concentration of coelenterazine in the final reaction mixture was 2.2 μM while the concentration of luciferase enzymes was 50 nM. The obtained luminescence kinetic data were fitted to an exponential decay or a logistic model using Origin 6.1 (OriginLab, USA) and the value of half-life $t_{1/2}$, defined as the time when the initial luminescence signal decreased to 50 %, was calculated.

Representative luminescence curves showing the stability of bioluminescence emission signal are shown in Fig. **11**. The kinetic traces showed that the flash-type bioluminescence signal, typical for RLuc and RLuc8, was changed and the newly constructed proteins, AncFT, AncFT-D160A, and AncFT-L14, exhibited the glow-type bioluminescence signal with a significantly prolonged and stable light emission (see Table IV). The half-life of the AncFT, AncFT-D160A, and AncFT-L14 bioluminescence signal decays increased more than 100-fold compared to any of the tested and previously reported Renilla-luciferases, including RLuc, Rluc8, and Super RLuc8.

Moreover, thanks to the significantly increased half-life of AncFT and AncFT-L14, their luminescence intensities remained the highest of all the tested variants after 5 minutes, confirming their usefulness for long-term bioluminescence measurements.

TABLE IV

| Luciferase enzyme | Half-life ($t_{1/2}$) [min] |
|---|---|
| RLuc | $0.274 \pm 0.001$ |
| RLuc8 | $0.100 \pm 0.001$ |
| Super RLuc8 | $0.237 \pm 0.004$ |
| AncFT | $18.6 \pm 0.1$ |
| AncFT-L14 | $3.86 \pm 0.04$ |
| AncFT-D160A | $1.84 \pm 0.02$ |

**Example 8. Steady-state kinetic analysis and determination of affinities towards substrate and product**

Solid coelenterazine was dissolved in ice-cold ethanol and stored under nitrogen atmosphere in dark glass vials at -20 °C. Before each measurement, concentration and maintained quality of the ethanol stock solutions was verified spectrophotometrically. A buffer solution of coelenterazine was prepared by mixing an appropriate volume of the ethanol stock solution with 100 mM phosphate buffer pH 7.5 immediately before the measurement. Tested luciferase enzyme samples were dissolved in the same 100 mM phosphate buffer pH 7.5. The enzymatic reaction was initiated inside the microplate reader FLUOstar OPTIMA (BMG Labtech, Germany) by automatic addition of 225 μL buffer solution of coelenterazine into a microplate well containing 25 μL of enzyme solution. The resulting bioluminescence activity traces were collected at 37 °C until the luminescence intensity decreased to less than 0.5 % of its maximal measured value to assure the full conversion of coelenterazine. Each luminescence trace was measured in 3 repetitions. The concentration of coelenterazine in the final reaction mixture ranged from 0.2 to 7.9 μM while the concentration of luciferase enzymes ranged from 17 to 640 nM.

An updated protocol applying new standards for collecting and fitting steady-state kinetic data was used. Unlike the classical initial velocity analysis, which requires a sophisticated luminometer calibration and quantum yield evaluation, luminescence data were recorded when letting the reaction proceed to completion beyond the initial linear phase. The recorded luminescence traces (rate vs. time) were transformed to reaction progress curves corresponding to cumulative luminescence in time. The transformed kinetic data (product vs. time) were fitted globally with the KinTek Explorer (KinTek Corporation, USA). The software allows for the input of a given kinetic model via a simple text description, and the program then derives the differential equations needed for numerical integration automatically.

Numerical integration of rate equations searching a set of kinetic parameters that produce a minimum $X^2$ value was performed using the Bulirsch-Stoer algorithm with adaptive step size, and nonlinear regression to fit data was based on the Levenberg-Marquardt method. To account for fluctuations in experimental data, enzyme or substrate concentrations were slightly adjusted ($\pm$ 5 %) to derive best fits. Residuals were normalized by sigma values for each data point. The standard error was calculated from the covariance matrix during nonlinear regression. In addition to standard error values, more rigorous analysis of the variation of the kinetic parameters was accomplished by confidence contour analysis by using FitSpace Explorer (KinTek Corporation, USA). In these analyses, the lower and upper limits for each parameter were derived from the confidence contour obtained from setting $X^2$ threshold at 0.9. The scaling factor, relating luminescence signal to product concentration, was applied as one of the fitted parameters, well constrained by end-point levels of kinetic traces recorded at particular substrate concentrations. Depletion of available substrate after the reaction was ensured by repeated injection of the fresh enzyme.

The steady-state model, described by Equation (4), was used to obtain the values of turnover number $k_{cat}$, Michaelis constant $K_m$, and equilibrium dissociation constant for enzyme-product complex $K_p$. A conservative estimate for diffusion-limited substrate and product binding $k_1$ and $k_{-3}$ (100 $\mu$M$^{-1}$.s$^{-1}$) was used as a fixed value to mimic rapid equilibrium assumption. An alternative form of the model, described by Equation (5), was used to obtain estimates of $k_{cat}/K_m$ directly by setting $k_{-1}$ = o.

$$E + S \underset{k_{-1}}{\overset{k_{+1} = 100\ \mu M^{-1}.s^{-1}}{\rightleftarrows}} E.S \xrightarrow{k_{+2}} E.P \underset{k_{-3} = 100\ \mu M^{-1}.s^{-1}}{\overset{k_{+3}}{\rightleftarrows}} E + P \qquad (4)$$

$k_{cat} = k_{+2}$
$K_m = k_{-1}/100$
$K_p = k_{+3}/100$

$$E + S \underset{k_{-1} = 0\ s^{-1}}{\overset{k_{+1}}{\rightleftarrows}} E.S \xrightarrow{k_{+2}} E.P \underset{k_{-3} = 100\ \mu M^{-1}.s^{-1}}{\overset{k_{+3}}{\rightleftarrows}} E + P \qquad (5)$$

$k_{cat}/K_m = k_{+1}$

Representative full conversion curves of the cumulative luminescence signal over time at different coelenterazine substrate concentrations together with their best fits are shown in Fig. **12.** The derived steady-state parameters (see Table V) quantitatively described the improved kinetics and thermodynamics of the AncFT, AncFT-D160A, and AncFT-L14 enzymes. Although the three proteins were limited by lower turnover numbers $k_{cat}$, their overall catalytic efficiency, given by the value $k_{cat}/K_m$, was at the level of RLuc and RLuc8. $K_m$ values of AncFT, AncFT-D160A, and AncFT-L14 were approximately 10 to 65-fold lower compared to RLuc and RLuc8, indicating that saturation of these enzymes occurred at significantly low concentrations of the substrate, allowing to use decreased amount of coelenterazine in luminescence assays while keeping the maximal bioluminescence activity. The variant AncFT-L14 exhibited the lowest $K_m$ value of all the tested proteins, making this protein the most suitable coelenterazine-utilizing luciferase for practical applications where a minimal coelenterazine concentration is essential while retaining the full enzyme saturation and the maximal bioluminescence activity.

Table V further shows the affinity towards the generated product coelenteramide, described by the constant $K_p$. Comparable values of $K_p$ suggested similar affinities towards the product for all the tested variants. However, 10-fold decreased ratio of $K_m/K_p$ clearly illustrates significantly higher preference of the substrate over the product and significantly suppressed product inhibition effect in the case of AncFT, AncFT-D160A, and AncFT-L14, explaining the glow-type bioluminescence signal with the prolonged half-life. The variant AncFT-L14 showed the lowest $K_m/K_p$ ratio of all the tested proteins, concluding that AncFT-L14 has the most significantly suppressed product inhibition and possesses the highest preference of the substrate over the product.

TABLE V

| Luciferase enzyme | $K_m$ [$\mu$M] | $k_{cat}$ [S$^{-1}$] | $K_p$ [$\mu$M] | $k_{cat}/K_m$ [$\mu$M$^{-1}$.s$^{-1}$] | $K_m/K_p$ |
|---|---|---|---|---|---|
| RLuc[a] | 2.9 $\pm$ 1.0 | 3.9 $\pm$ 0.4 | n.d. | 1.3 $\pm$ 0.5 | n.d. |
| RLuc8 | 1.5 $\pm$ 0.1 | 4.7 $\pm$ 0.1 | 1.18 $\pm$ 0.05 | 3.0 $\pm$ 0.1 | 1.3 $\pm$ 0.1 |
| Super RLuc8 | 5.8 $\pm$ 0.1 | 6.49 $\pm$ 0.05 | 0.92 $\pm$ 0.01 | 1.1 $\pm$ 0.1 | 6.3 $\pm$ 0.1 |
| AncFT | 0.064 $\pm$ 0.001 | 0.111 $\pm$ 0.001 | 0.5 $\pm$ 0.1 | 1.6 $\pm$ 0.2 | 0.13 $\pm$ 0.03 |
| AncFT-L14 | 0.044 $\pm$ 0.002 | 0.168 $\pm$ 0.001 | 0.55 $\pm$ 0.03 | 3.8 $\pm$ 0.1 | 0.08 $\pm$ 0.01 |

(continued)

| Luciferase enzyme | $K_m$ [μM] | $k_{cat}$ [S$^{-1}$] | $K_p$ [μM] | $k_{cat}/K_m$ [μM$^{-1}$.s$^{-1}$] | $K_m/K_p$ |
|---|---|---|---|---|---|
| AncFT-D160A | 0.148 ± 0.006 | 0.339 ± 0.001 | 0.23 ± 0.01 | 2.3 ± 0.1 | 0.64 ± 0.04 |

[a] Loening et al., (2006). Protein Eng. Des. Sel. 19, 391-400. n.d. = not determined

## Example 9. Analysis of bioluminescence emission spectrum and determination of peak maximum wavelength

Bioluminescence emission spectra were measured at ambient temperature using the spectrofluorometer FluoroMax-4 (HORIBA, Japan). 1 mL of an enzyme solution in 100 mM potassium phosphate buffer pH 7.5 was added into a quartz cuvette and the enzymatic reaction was initiated by manual injection of 50 μL of ethanolic solution of coelenterazine followed by a quick mixing. The acquisition of luminescence emission spectra started after 10 seconds from the addition of the coelenterazine substrate. The resulting concentration of an enzyme in the reaction mixture ranged from 0.7 to 1.0 μM while the concentration of coelenterazine was approximately 30 μM. The data were collected for wavelengths from 350 to 700 nm with a step of 2 nm and each spectrum was measured in triplicates. The obtained spectrum was normalized relative to the value of the highest peak for each of the enzyme individually and the shape and positions of the luminescence emission peaks were compared.

Normalized representative bioluminescence emission spectra of the tested coelenterazine-utilizing luciferases are summarized in Fig. **13.** The determined wavelengths of the emission peak maxima (Table VI) showed that all the newly generated luciferases (AncFT, AncFT-L14, and AncFT-D160A) exhibited a blue-shifted bioluminescence emission compared to the *Renilla* luciferase and their mutants. While the shift was not very significant for AncFT and AncFT-L14 (15-20 nm), in the case of AncFT-D160A, the spectrum was strongly blue-shifted to the near-UV region with the emission peak of 390 nm. The blue-shifted luciferase protein AncFT-D160A will find a practical usage in the dual luciferase assays where two different processes need to be monitored simultaneously by using two different luciferases with distinct emission peaks. Importantly, compared to a standard dual assay, which uses a combination of *Renilla* and firefly luciferases, the usage of RLuc/AncFT-D160A or AncFT/AncFT-D160A combination will require only a single substrate to obtain a dual signal directly. Advantageously, the AncFT/AncFT-D160A combination would yield a "dual glow assay" as the stable glow-type bioluminescence is maintained for both proteins, as shown in Example 7.

TABLE VI

| Luciferase enzyme | Peak maximum wavelength ($\lambda_{max}$) [nm] |
|---|---|
| RLuc | 470 |
| RLuc8 | 478 |
| Super RLuc8 | 540 |
| AncFT | 454 |
| AncFT-L14 | 456 |
| AncFT-D160A | 390 |

## Example 10. Analysis of secondary structure and temperature stability and determination of melting temperature

Circular dichroism spectra, providing information about the secondary structure composition and correct folding of proteins, were recorded at 20 °C using a spectropolarimeter Chirascan (Applied Photophysics, United Kingdom). Data were collected from 185 to 260 nm at 100 nm/min with a 1 second response time and a 1 nm bandwidth in a 0.1 cm quartz cuvette. Each spectrum was measured in five repetitions and was subsequently corrected for the absorbance of the buffer. The circular dichroism data were expressed in terms of the mean residue ellipticity $\Theta_{MRE}$, calculated according to Equation (6) where $\Theta_{obs}$ is the observed ellipticity in degrees, $M_w$ is the protein molecular weight, n is the number of residues, 1 is the cell path length, c is the protein concentration (in mg/mL) and the factor of 100 originates from the conversion of the molecular weight to mg/dmol.

$$\Theta_{MRE} = \frac{\Theta_{obs} \cdot M_w \cdot 100}{n \cdot c \cdot l} \qquad (6)$$

Thermal unfolding was studied using NanoDSF Prometheus (NanoTemper, Germany) by monitoring tryptophan fluorescence over the temperature range of 20 to 90 °C, at a heating rate of 1 °C/min. The melting temperatures ($T_{onset}$

and $T_m$) were evaluated directly by ThermControl v2.0.2.

Representative circular dichroism spectra of the tested luciferase enzymes are shown in Fig. **14.** All the spectral curves retained comparable shapes and trends, showing that all the newly created luciferase proteins exhibited a very similar secondary structure compositions and the overall fold.

Representative melting curves of tryptophan fluorescence changes with increasing temperature are shown in Fig. **15.** The determined values of $T_{onset}$ and $T_m$ (see Table VII) confirmed that the luciferase enzymes AncFT, AncFT-D160A, and AncFT-L14 exhibited thermostabilities that were high enough for their application as reporters and bioimaging agents at the physiological temperature. The thermostability was increased compared to the original luciferase RLuc.

TABLE VII

| Luciferase enzyme | $T_{onset}$ [°C] | $T_m$ [°C] |
|---|---|---|
| RLuc | $36.3 \pm 0.2$ | $43.7 \pm 0.1$ |
| RLuc8 | $35.2 \pm 0.1$ | $45.0 \pm 0.1$ |
| Super RLuc8 | $39.1 \pm 0.3$ | $50.1 \pm 0.2$ |
| AncHLD-RLuc | $57.4 \pm 0.1$ | $64.9 \pm 0.1$ |
| AncFT | $50.5 \pm 0.1$ | $56.6 \pm 0.1$ |
| AncFT-L14 | $39.8 \pm 0.2$ | $47.0 \pm 0.1$ |
| AncFT-D160A | $51.5 \pm 0.1$ | $57.9 \pm 0.1$ |

**Example 11. Kinetic analysis of the importance of transplanted fragments for the substrate binding efficiency**

The measurement was performed at 15 °C in 100 mM potassium phosphate buffer pH 7.5. Rapid mixing of the components was achieved using the Stopped-Flow SFM 3000 combined with the MOS-500 spectrometer (BioLogic, France). The reaction was initiated by mixing 75 $\mu$L of an enzyme with 75 $\mu$L of coelenterazine and then monitored by the change of native tryptophan fluorescence (295 nm excitation, 340 $\pm$ 13 nm emission). The fluorescence changes were caused by the quenching effect of the coelenterazine substrate upon its binding by an enzyme. The experiment was designed in two different modes, varying either the concentration of the coelenterazine substrate or the concentration of an enzyme. Each kinetic trace was measured in seven consecutive replicates and averaged. The concentration of coelenterazine in the final reaction mixture ranged from 0.9 to 90 $\mu$M while the concentration of luciferase enzymes ranged from 0.4 to 94 $\mu$M. The drop of the initial fluorescence level caused by the non-specific interactions of coelenterazine was corrected based on experiments with bovine serum albumin, which exhibited the same initial decrease of the fluorescence signal.

The collected double-exponential kinetic traces were fitted globally with the KinTek Explorer (KinTek Corporation, USA). The software allows for the input of a given kinetic model via a simple text description, and the program then derives the differential equations needed for numerical integration automatically. Numerical integration of rate equations searching a set of kinetic parameters that produce a minimum $X^2$ value was performed using the Bulirsch-Stoer algorithm with adaptive step size, and nonlinear regression to fit data was based on the Levenberg-Marquardt method. Residuals were normalized by sigma values for each data point. The standard error was calculated from the covariance matrix during nonlinear regression. In addition to standard error values, more rigorous analysis of the variation of the kinetic parameters was accomplished by confidence contour analysis by using FitSpace Explorer (KinTek Corporation, USA). In these analyses, the lower and upper limits for each parameter were derived from the confidence contour obtained from setting $X^2$ threshold at 0.95.

The time-course of the fluorescence quenching upon substrate binding was described with Equation (7) according to the induced-fit binding mechanism and the mechanism was fit directly to the collected kinetic data to obtain the values of elementary rate constants describing the substrate binding process. The quality of the best fits was compared in terms of $X^2$ values, "chi-by-eye" assessment, and confidence contour analysis assessing constraint and independency of the determined parameters which confirmed that the obtained values are well-defined by the collected kinetic data.

$$ E \ + \ S \ \underset{k_{-1}}{\overset{k_1}{\rightleftharpoons}} \ E.S \ \underset{k_{-2}}{\overset{k_2}{\rightleftharpoons}} \ E^*.S \qquad (7) $$

Representative pre-steady-state double-exponential tryptophan fluorescence kinetic curves at different coelenterazine substrate and luciferase enzyme concentrations, together with their best fits according to the induced-fit binding mechanism, are shown in Fig. **16.** The derived elementary rate constants describing the substrate binding process (see Table VIII) showed that while no conformational flexibility and only a simple one-step binding could be observed for the rigid structure of AncHLD-RLuc, the binding mechanism and overall binding efficiency of AncFT was very similar to RLuc8. Since the subsequent chemical transformation is slower in the case of AncFT, observable as a lower $k_{cat}$ value determined by steady-state kinetics (see Table V), the AncFT enzyme can be saturated by the coelenterazine substrate more easily and the maximal overall catalytic efficiency of AncFT is reached at a lower substrate concentration. Such observation confirms that AncFT is suitable for bioluminescence measurements at experimental conditions where low coelenterazine concentration is desirable.

TABLE VIII

| Luciferase enzyme | $k_{+1}$ [$\mu M^{-1}.s^{-1}$] | $k_{-1}$ [$s^{-1}$] | $k_{+2}$ [$s^{-1}$] | $k_{-2}$ [$s^{-1}$] |
|---|---|---|---|---|
| AncHLD-RLuc | $0.003 \pm 0.003$ | $0.118 \pm 0.071$ | n.a. | n.a. |
| RLuc8 | $2.35 \pm 0.04$ | $305 \pm 5$ | $26.6 \pm 1.1$ | $23.4 \pm 0.2$ |
| AncFT | $5.21 \pm 0.07$ | $367 \pm 5$ | $31.9 \pm 1.1$ | $27.5 \pm 0.4$ |

n.a.= not applicable

**Example 12. Equilibrium analysis of coelenterazine binding and determination of ligands affinities**

The experiments were performed under strictly anaerobic conditions in order to prevent enzymatic oxidation of interacting ligands. To do so, all the equipment and material, including water, buffer, pipettes, tips, syringes, microplates, and sealing, were made anaerobic by incubating them overnight in the anaerobic glovebox Belle MR2 (Belle Technology, UK). The box was filled with ultra-high purity (UHP) nitrogen atmosphere containing the residual oxygen concentration of 2.3 ppm, as determined by the oxygen meter O2M-3 (Belle Technology, UK). Liquids were aliquoted to the volume of maximally 50 mL and were stirred overnight in the box to release any dissolved oxygen. Two hours prior to the experiment, a closed bottle with an evacuated lyophilized enzyme (oxygen depleted) was put into the glovebox, dissolved with anaerobic water, and incubated to remove any remaining oxygen possibly dissolved in the solution. Similarly, the stock solution of coelenterazine or coelenteramide in ethanol, which was long-term stored in a freezer under the nitrogen atmosphere without oxygen, was put into the glovebox in a closed bottle. A small aliquot of coelenterazine/coelenteramide was transferred into a new empty bottle and kept open for about 20 minutes to remove the remaining dissolved oxygen. After the incubation, a few microliters of an enzyme were mixed with the same amount of coelenterazine, and the mixture was visually inspected for any possible bioluminescence. If no light was observed and the mixture remained yellow even after a minute, all the reagents were ready for anaerobic measurements.

In the anaerobic glovebox, 200 $\mu L$ of a differently concentrated enzyme was put into a separate well in a microplate and into each of these wells, 5 $\mu L$ of coelenterazine or coelenteramide stock solution was added. As a blank, 200 $\mu L$ of the enzyme was mixed with 5 $\mu L$ of ethanol for each enzyme concentration. The resulting concentration of an enzyme in the mixture ranged from 0 to 400 $\mu M$ while the resulting concentration of coelenterazine and coelenteramide was approximately 15 and 14 $\mu M$, respectively. The incubation buffer was 100 mM potassium phosphate buffer pH 7.5. The microplate was thoroughly covered with an airtight UV transparent sealing to isolate wells from the surrounding environment and the plate was removed from the anaerobic glovebox. The extent of coelenterazine binding at each concentration was determined by measuring fluorescence spectra ranging from 480 to 580 nm upon excitation at 420 nm using the microplate spectrophotometer Varioskan LUX (Thermo Scientific, USA). Binding of coelenteramide was monitored by measuring fluorescence spectra ranging from 350 to 700 nm upon excitation at 330 nm. Excitation bandwidth was set to 5 nm, measurement time to 100 ms, and the temperature inside the instrument to 30 °C.

The obtained fluorescence spectra were visually inspected for characteristic peaks, and the value of the maximal fluorescence intensity $Fluo_{max}$ at these peaks was plotted against the enzyme concentration. The final value of the dissociation constant $K_d$ was determined by fitting the dependence with the hyperbolic Equation (8) using the software Origin 6.1 (OriginLab, USA).

$$\text{Fluo}_{max} = \frac{Fluo_{lim} \cdot [E]}{K_d + [E]} + Fluo_0 \qquad (8)$$

Representative emission fluorescence spectra and the changes of their intensities with increasing enzyme concentrations are provided in Fig. **17**. The dependence of emission peak intensities on enzyme concentrations are shown in Fig. **18**. The results showed a clear concentration dependence of fluorescence intensities with a hyperbolic trend, typical for saturation of a ligand by a protein. The derived dissociation constants $K_d$ for each enzymeligand pair (see Table IX) confirmed increased affinity of AncFT towards the coelenterazine substrate, allowing enzyme saturation at lower concentrations and reaching the maximal catalytic efficiency even under the conditions where low coelenterazine concentrations are needed. Table IX further confirms that AncFT exhibited markedly improved affinity towards the coelenterazine substrate over the coelenteramide product compared to RLuc8, as indicated by a lower $K_d$ ratio. Such result is in a good agreement with the steady-state kinetic measurements, concluding that the AncFT preference for the substrate over the product leads to a lower product inhibition effect, and therefore to a prolonged half-life of the luminescence signal stability.

TABLE IX

| Luciferase enzyme | Dissociation constant $K_d$ [$\mu$M] | | $K_d$ ratio substrate/product |
| --- | --- | --- | --- |
| | Coelenterazine substrate | Coelenteramide product | |
| RLuc8 | 37 ± 15 | 22 ± 2 | 1.7 ± 0.7 |
| AncFT | 12 ± 5 | 16 ± 2 | 0.7 ± 0.3 |

**Example 13. Confirmation of improved conformational flexibility by X-ray crystallography, HDX-MS, and MD simulations**

In order to confirm that transplantation of the L9-$\alpha$4 and L14 regions from RLuc to AncHLD-RLuc improved conformational flexibility, the newly constructed coelenterazine-utilizing luciferases AncFT and AncFT-L14 were analyzed by three more different approaches - X-ray crystallography, HDX-MS, and MD simulations. HDX-MS experiments and MD simulations were performed as previously described in Example 3 and Example 4, respectively. MD simulations were carried out on the crystal structures of RLuc8 (PDB 2PSF), AncHLD-RLuc (PDB 6G$_{75}$), and AncFT (PDB 6S$_{97}$) while the structure of AncFT-L14 was generated by homology modelling, using AncFT as a template.

Crystals of AncFT were obtained after optimization using a seeding technique at 20 °C after 2 days by mixing 0.5 $\mu$L of seed, 2 $\mu$L of AncFT (9.2 mg/mL), and 1 $\mu$L of reservoir solution (0.2 M sodium acetate, 0.1 M Tris pH 8.5, 19 % PEG 3350). The hanging-drop vapour diffusion technique in EasyXtal 15-well plates (Qiagen, Germany) was used. Crystals used for X-ray data collection were briefly transferred in reservoir solution supplemented with 20 % glycerol and flash-frozen in liquid nitrogen. Crystallographic data were collected at SLS beamline PXIII in Villigen (Switzerland).

The crystallographic data were processed and scaled using XDS (Kabsch, (2010). Acta Crystallogr. D Biol. Crystallogr. 66, 125-132.) and Aimless (Evans & Murshudov (2013). Acta Crystallogr. D Biol. Crystallogr. 69, 1204-1214.). Structures of AncFT and AncFT-L14 were solved by molecular replacement using AncHLD-RLuc (PDB 6G$_{75}$) as a search model with the help of Phaser (McCoy et al., (2007). J. Appl. Crystallogr. 40, 658-674.) as implemented in the Phenix package (Adams et al., (2011). Methods Struct. Proteomics. 55, 94-106.). Multiple cycles of automated refinement were performed in the phenix.refine program and manual model building was performed in Coot (Emsley et al., (2010). Acta Crystallogr. D Biol. Crystallogr. 66, 486-501.). The final models were validated using tools provided in Coot and Molprobity (Chen et al., (2010). Acta Crystallogr. D Biol. Crystallogr. 66, 12-21.). Graphical visualization of structural data was done using The PyMOL Molecular Graphics System, version 1.8 (Schrödinger, USA). Atomic coordinates and structure factors for AncFT were deposited in the Protein Data Bank under the PDB code 6S97.

The final crystal structure of AncFT is presented in Fig. **4** and the refinement statistics are provided in Table X. Crystallographic analysis of AncFT revealed a canonical $\alpha/\beta$-hydrolase fold similar to that of AncHLD-RLuc, but with some structural features typical of RLuc8. Most importantly, the active site cavity and the $\alpha$4 helix conformation are both more open than in AncHLD-RLuc, and even than in chain B of RLuc8. Chain A of RLuc8 has the most open conformation seen in any of the structures. HDX-MS experiments, as represented in Fig. **5**, showed that the deuteration pattern of AncFT was almost identical to that of AncHLD-RLuc, with differences occurring only in the transplanted region. Specifically, the end of the L9 loop was less extensively deuterated than in AncHLD-RLuc while the $\alpha$4-$\alpha$5' helices were more heavily deuterated. A similar trend was observed for RLuc8, providing experimental evidence that the mutant bearing the transplanted fragment mimics the cap domain dynamics of RLuc8. MD simulations of AncFT are provided in Fig. **6.** The results revealed that the B-factor profile of its cap domain closely resembled that for RLuc8. The highest B-factor values occur in the $\alpha$5' and $\alpha$4 helices, while the L9 and L14 loops have B-factors close to the average for the rest of the enzyme and the B-factors for the rest of the cap domain are lower. In case of AncFT-L14, an analogical dynamic profile could be observed with the exception of the transplanted loop L14 which exhibited significantly higher flexibility compared to AncFT. Collectively, the results obtained by X-ray, HDX-MS, and MD all confirmed that the transplanted region was important for effective binding of the bulky coelenterazine substrate and that transplantation of the flexible elements increased conformational flexibility of these regions, allowing efficient catalysis and bioluminescence activity.

TABLE X

| Protein | AncFT |
|---|---|
| Space group | $P3_121$ |
| Cell parameters | |
| $a$, $b$, $c$ [Å] | 87.556, 87.556, 102.125 |
| $\alpha$, $\beta$, $\gamma$ [°] | 90, 90,120 |
| Resolution range [Å] | 43.78-1.953 (2.023-1.953) |
| Total reflections | 669493 (66368) |
| Number of unique reflections | 33335 (3277) |
| Completeness [%] | 99.9 (99.3) |
| Multiplicity | 20.1 (20.3) |
| I/$\sigma$ [I] | 19.9 (2.9) |
| $R_{merge}$ | 14.08 (129.2) |
| $CC_{1/2}$ | 0.999 (0.871) |
| Refinement statistics | |
| Resolution [Å] | 43.78-1.953 |
| No. of reflections | 33321 (3274) |
| $R_{work}$ [%] | 29.1** |
| $R_{free}$ [%] | 33.0** |
| RMSD bond lengt [Å] | 0.007 |
| RMSD angle [°] | 0.92 |
| Ramachandran favored | 95.9 |
| Ramachandran allowed | 3.8 |
| Ramachandran outliers | 0.3 |
| Contents of asymmetric unit | |
| No. of protein atoms in AU | 2405 |
| No. of water molecules in AU | 140 |
| No. of ligand atoms | - |
| Mean B values | |
| Protein [Å$^2$] | 28.4 |
| Ligands [Å$^2$] | - |
| Waters [Å$^2$] | 32.7 |
| PDB code | 6S$_{97}$ |

** Higher $R_{work}$ and $R_{free}$ values are due to the void (uninterpretable) electron density present in the asymmetric unit

**Example 14. Confirmation of improved bioluminescence signal stability in a mammalian cells lysate**

NIH/$_3$T$_3$ mouse fibroblast cells (ATCC® CRL-1658™) were transfected according to the manufacturer protocol using Lipofectamine 2000 (Thermo Fisher, USA) with pcDNA3.1(+) plasmids containing the genes of luciferases codon-optimized for expression in mammalian cells (Gene Art, Thermo Fisher, US). Cells were lysed 24 hours after the transfection, and the bioluminescence signal in the lysate was measured with a microplate reader FLUOstar Omega (BMG Labtech, Germany) using the commercial *Renilla* Luciferase Assay System (Promega, USA) and also using an in-house prepared assay mixture composed of 100 mM potassium phosphate buffer pH 7.5 with 4.5 $\mu$M coelenterazine (final concentration). Cells transfected with an empty pcDNA3.1(+) plasmid were used as a negative control. The measurements were done in three independent replicates.

Representative micrographs of the transfected cells expressing a gene of each of the tested luciferase enzymes are provided in Fig. **19**. The snapshots showed that the morphology of the cells was not disrupted by the transfection process and that the viability of the cells was maintained. Luminescence curves obtained after mixing the cell lysate with coelenterazine are shown in Fig. **20**. Results consistent with the measurements of luciferase proteins purified from a bacterial culture (Fig. **11**) could be observed. The comparison clearly distinguished the glow-type stable luminescence signal of AncFT and AncFT-L14 while the signal of the other *Renilla*-type luciferases exhibited a typical flash of luminescence with a fast decrease and no steadiness. The highly stable glow-type bioluminescence property of AncFT and AncFT-L14 thus persisted even after heterologously expressed in mammalian mouse fibroblast cells. The bioluminescence signal of both AncFT and AncFT-L14 remained at the level of more than 80 % of its maximum for at least 60 minutes. This result indicates that AncFT and AncFT-L14 could be used as reporter proteins for in *vivo*

**Claims**

1. A coelenterazine-utilizing polypeptide having at least 85 % sequence identity to SEQ ID NO: 6, or at least 85 % sequence identity to SEQ ID NO: 8 containing at least an amino acid substitution D160A.

2. The coelenterazine-utilizing polypeptide according to Claim **1**, wherein the polypeptide has SEQ ID NO: 6 or SEQ ID NO: 8.

3. The coelenterazine-utilizing polypeptide according to Claim **1** or Claim **2,** wherein a part of the sequence with SEQ ID NO: 6 or SEQ ID NO: 8 is LVKGGKP.

4. The coelenterazine-utilizing polypeptide according to Claim **3,** wherein the polypeptide has SEQ ID NO: 7 or SEQ ID NO: 9.

5. A polynucleotide having a sequence encoding a polypeptide according to any of Claims **1** to **4.**

6. A polynucleotide according to Claim **5**, wherein the sequence is selected from a group containing SEQ ID NO: 2, SEQ ID NO: 3, SEQ ID NO: 4, SEQ ID NO: 5.

7. A polynucleotide encoding a fusion protein composed of a coelenterazine-utilizing polypeptide according to Claims **1** to **4** and at least one fusion partner.

8. A vector containing the polynucleotide according to Claim **5** or Claim **6**.

9. An expression cassette containing the polynucleotide according to Claim **5** or Claim **6.**

10. A cell containing the vector according to Claim **8** or the expression cassette according to Claim **9.**

11. A kit containing a vector according to Claim **8** or an expression cassette according to Claim **9.**

12. A kit containing a polypeptide according to any of Claims **1** to **4.**

13. A method of producing and isolating polypeptides according to any of Claims **1** to **4,** including growing a cell according to Claim **10** wherein the polypeptides are expressed, followed by isolating the polypeptides that are substantially free of other proteins.

14. Use of polypeptides according to any of Claims **1** to **4** as bioluminescence reporters.

15. Use of polypeptides according to any of Claims **1** to **4** as bioimaging agents.

Fig. 1

Fig. 2

A

kDa   M   RLuc   Anc
116.0
66.2
45.0
35.0
25.0
18.4

B

C

----RLuc (Tm = 43.7 °C)
-- --AncHLD-RLuc (Tm = 64.9 °C)

D

----RLuc   -- --AncHLD-RLuc

Fig. 3

Fig. 4

Fig. 5

Fig. 6

Fig. 7

```
AncHLD-RLuc    1 ---MVSASQRTTSTATGDEWWAKCKQVDVLDSEMSYYDSDPGKHKNT 44
AncFT          1 ---MVSASQRTTSTATGDEWWAKCKQVDVLDSEMSYYDSDPGKHKNT 44
AncFT-D160A    1 ---MVSASQRTTSTATGDEWWAKCKQVDVLDSEMSYYDSDPGKHKNT 44
AncFT-L14      1 ---MVSASQRTTSTATGDEWWAKCKQVDVLDSEMSYYDSDPGKHKNT 44
RLuc           1 MTSKVYDPEQRKRMITGPQWWARCKQMNVLDSFINYYDSEK-HAENA 46
RLuc8          1 MTSKVYDPEQRKRMITGPQWWARCKQMNVLDSFINYYDSEK-HAENA 46
Super RLuc8    1 MTSKVYDPEQRKRMITGPQWWARCKQMNVLDSFINYYDSEK-HAENA 46

AncHLD-RLuc   45 VIFLHGNPTSSYLWRNVIPHVEPLARCLAPDLIGMGKSGKLPNHSYR 91
AncFT         45 VIFLHGNPTSSYLWRNVIPHVEPLARCLAPDLIGMGKSGKLPNHSYR 91
AncFT-D160A   45 VIFLHGNPTSSYLWRNVIPHVEPLARCLAPDLIGMGKSGKLPNHSYR 91
AncFT-L14     45 VIFLHGNPTSSYLWRNVIPHVEPLARCLAPDLIGMGKSGKLPNHSYR 91
RLuc          47 VIFLHGNAASSYLWRHVVPHIEPVARCIIPDLIGMGKSGKSGNGSYR 93
RLuc8         47 VIFLHGNATSSYLWRHVVPHIEPVARCIIPDLIGMGKSGKSGNGSYR 93
Super RLuc8   47 VIFLHGNATSSYLWRHVVPHIEPVARCIIPDLIGMGKSGKSGNGSYR 93

AncHLD-RLuc   92 FVDHYRYLSAWFDSVNLPEKVTIVCHDWGSGLGFHWCNEHRDRVKGI 138
AncFT         92 FVDHYRYLSAWFDSVNLPEKVTIVCHDWGSGLGFHWCNEHRDRVKGI 138
AncFT-D160A   92 FVDHYRYLSAWFDSVNLPEKVTIVCHDWGSGLGFHWCNEHRDRVKGI 138
AncFT-L14     92 FVDHYRYLSAWFDSVNLPEKVTIVCHDWGSGLGFHWCNEHRDRVKGI 138
RLuc          94 LLDHYKYLTAWFELLNLPKKIIFVGHDWGACLAFHYSYEHQDIKIAI 140
RLuc8         94 LLDHYKYLTAWFELLNLPKKIIFVGHDWGAALAFHYAYEHQDRIKAI 140
Super RLuc8   94 LLDHYKYLTAWFELLNLPKKIIFVGHDWGSALAFHYAYEHQDRIKAI 140

AncHLD-RLuc  139 VHMESVVSPLKGWESFPETARDIFQALRSEAGEEMVLKKNFFIERLL 185
AncFT        139 VHMESVVDVIESWDEWPDIEEDI-ALIKSEAGEEMVLKKNFFIERLL 184
AncFT-D160A  139 VHMESVVDVIESWDEWPDIEEAI-ALIKSEAGEEMVLKKNFFIERLL 184
AncFT-L14    139 VHMESVVDVIESWDEWPDIEEDI-ALIKSEAGEEMVLKKNFFIERLL 184
RLuc         141 VHAESVVDVIESWDEWPDIEEDI-ALIKSEEGEKMVLENNFFVETML 186
RLuc8        141 VHMESVVDVIESWDEWPDIEEDI-ALIKSEEGEKMVLENNFFVETVL 186
Super RLuc8  141 VHMESVVDVIESWKNWPDIEEEL-ALIKSEEGEKMVLENNFFVETVL 186

AncHLD-RLuc  186 PSSIMRKLSEEEMDAYREPFVEPGESRRPTLTWPREIPIKGDGPEDV 232
AncFT        185 PSSIMRKLSEEEMDAYREPFVEPGESRRPTLTWPREIPIKGDGPEDV 231
AncFT-D160A  185 PSSIMRKLSEEEMDAYREPFVEPGESRRPTLTWPREIPIKGDGPEDV 231
AncFT-L14    185 PSSIMRKLSEEEMDAYREPFVEPGESRRPTLTWPREIPLVKGGKPDV 231
RLuc         187 PSKIMRKLEPEEFAAYLEPFKEKGEVRRPTLSWPREIPLVKGGKPDV 233
RLuc8        187 PSKIMRKLEPEEFAAYLEPFKEKGEVRRPTLSWPREIPLVKGGKPDV 233
Super RLuc8  187 PSVIMRKLEPEEFAAYLEPFKEKGEVRRPTLSWPREIPLVKGGKPDV 233

AncHLD-RLuc  233 IEIVKSYNKWLSTSKDIPKLFINADPGFFSNAIKKVTKNWPNQKTVT 279
AncFT        232 IEIVKSYNKWLSTSKDIPKLFINADPGFFSNAIKKVTKNWPNQKTVT 278
AncFT-D160A  232 IEIVKSYNKWLSTSKDIPKLFINADPGFFSNAIKKVTKNWPNQKTVT 278
AncFT-L14    232 IEIVKSYNKWLSTSKDIPKLFINADPGFFSNAIKKVTKNWPNQKTVT 278
RLuc         234 VQIVRNYNAYLRASDDLPKMFIESDPGFFSNAIVEGAKKFPNTEFVK 280
RLuc8        234 VQIVRNYNAYLRASDDLPKLFIESDPGFFSNAIVEGAKKFPNTEFVK 280
Super RLuc8  234 VQIVRNYNAYLRASDDLPKLFIESDPGWFSNAIIEGAKKFPNTEFVK 280

AncHLD-RLuc  280 VKGLHFLQEDSPEEIGEAIADFLNELTK---         307
AncFT        279 VKGLHFLQEDSPEEIGEAIADFLNELTK---         306
AncFT-D160A  279 VKGLHFLQEDSPEEIGEAIADFLNELTK---         306
AncFT-L14    279 VKGLHFLQEDSPEEIGEAIADFLNELTK---         306
RLuc         281 VKGLHFSQEDAPDEMGKYIKSFVERVLKNEQ         311
RLuc8        281 VKGLHFLQEDAPDEMGKYIKSFVERVLKNEQ         311
Super RLuc8  281 VKGLHFLQEDAPDEMGKYIKSFVERVLKNEQ         311
```

Fig. 8

Fig. 9

B

Fig. **9** (continue)

Fig. 9 (continue)

Fig. 10

Fig. 11

Fig. **12**

Fig. 13

Fig. 14

Fig. 15

Fig. 16

Fig. 17

Fig. 18

Fig. **19**

Fig. **20**

Europäisches
Patentamt

European
Patent Office

Office européen
des brevets

# EUROPEAN SEARCH REPORT

**Application Number**

EP 21 19 1447

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SCHENKMAYEROVA ANDREA ET AL: "Engineering the protein dynamics of an ancestral luciferase", NATURE COMMUNICATIONS, vol. 12, no. 1, 14 June 2021 (2021-06-14), XP055885815, DOI: 10.1038/s41467-021-23450-z Retrieved from the Internet: URL:https://www.nature.com/articles/s41467-021-23450-z.pdf> | 1,5,7-15 | INV. C12N9/02 C12N15/63 C12Q1/66 |
| A | * abstract; figures 4,5 * ----- | 2-4,6 | |
| A | LOENING ANDREAS MARKUS ET AL: "Consensus guided mutagenesis of Renilla luciferase yields enhanced stability and light output", PROTEIN ENGINEERING, DESIGN AND SELECTION, OXFORD JOURNAL, LONDON, GB, vol. 19, no. 9, 1 September 2006 (2006-09-01), pages 391-400, XP002524403, ISSN: 1741-0126, DOI: 10.1093/PROTEIN/GZL023 [retrieved on 2006-07-20] * table 1 * ----- | 1-15 | TECHNICAL FIELDS SEARCHED (IPC) C12N C40B G01N C12Q |
| A | LOENING A M ET AL: "Crystal Structures of the Luciferase and Green Fluorescent Protein from Renilla reniformis", JOURNAL OF MOLECULAR BIOLOGY, ACADEMIC PRESS, UNITED KINGDOM, vol. 374, no. 4, 7 December 2007 (2007-12-07), pages 1017-1028, XP026865862, ISSN: 0022-2836, DOI: 10.1016/J.JMB.2007.09.078 [retrieved on 2007-12-07] * abstract * ----- -/-- | 1-15 | |

The present search report has been drawn up for all claims

| Place of search | Date of completion of the search | Examiner |
|---|---|---|
| Munich | 1 February 2022 | Griesinger, Irina |

**page 1 of 2**

Europäisches
Patentamt

European
Patent Office

Office européen
des brevets

# EUROPEAN SEARCH REPORT

Application Number

EP 21 19 1447

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | RODA A ET AL: "Biotechnological applications of bioluminescence and chemiluminescence", TRENDS IN BIOTECHNOLOGY, ELSEVIER PUBLICATIONS, CAMBRIDGE, GB, vol. 22, no. 6, 1 June 2004 (2004-06-01), pages 295-303, XP004510121, ISSN: 0167-7799, DOI: 10.1016/J.TIBTECH.2004.03.011 * abstract * | 1-15 | |

-----

| | | | TECHNICAL FIELDS SEARCHED (IPC) |
|---|---|---|---|

The present search report has been drawn up for all claims

| Place of search | Date of completion of the search | Examiner |
|---|---|---|
| Munich | 1 February 2022 | Griesinger, Irina |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

............................................................................
& : member of the same patent family, corresponding
  document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- LIU ; ESCHER. *Gene,* 1999, vol. 237, 153-159
- LOENING et al. *Protein Eng. Des. Sel.,* 2006, vol. 19, 391-400
- LOENING ; WU ; GAMBHIR. *Nat. Methods.,* 2007, vol. 4, 641-643
- RAHNAMA. *Enzyme Microb. Technol.,* 2017, vol. 96, 60-66
- CHALOUPKOVA et al. *ACS Catal,* 2019, vol. 9, 4810-4823
- ALTSCHUL et al. *Nucleic Acids Res,* 1997, vol. 25, 3389-3402
- NCBI RESOURCE COORDINATORS. *Nucleic Acids Res,* 2013, vol. 41, D8-D20
- FRICKEY ; LUPAS. *Bioinformatics,* 2004, vol. 20, 3702-3704
- LI ; GODZIK. *Bioinformatics,* 2006, vol. 22, 1658-1659
- EDGAR. *BMC Bioinformatics,* 2004, vol. 5, 113
- HALL. *Nucleic Acids Symp. Ser.,* 1999, vol. 41, 95-98
- GUINDON et al. *Syst. Biol.,* 2010, vol. 59, 307-321
- LE ; GASCUEL. *Mol. Biol. Evol.,* 2008, vol. 25, 1307-1320
- ABASCAL et al. *Bioinformatics,* 2005, vol. 21, 2104-2105
- GUINDON ; GASCUEL. *Syst. Biol.,* 2003, vol. 52, 696-704
- HORDIJK ; GASCUEL. *Bioinformatics,* 2005, vol. 21, 4338-4347
- GASCUEL. *Mol. Biol. Evol.,* 1997, vol. 14, 685-695
- CHOVANCOVA et al. *Proteins,* 2007, vol. 67, 305-316
- KOSHI ; GOLDSTEIN. *J. Mol. Evol.,* 1996, vol. 42, 313-320
- YANG. *Mol. Biol. Evol.,* 2007, vol. 24, 1586-1591
- HANSON-SMITH ; KOLACZKOWSKI ; THORNTON. *Mol. Biol. Evol.,* 2010, vol. 27, 1988-1999
- FITCH. *Syst. Zool.,* 1971, vol. 20, 406-416
- FORTOVA et al. *Biochimie,* 2013, vol. 95, 2091-2096
- BATTYE et al. *Acta Crystallogr. D Biol. Crystallogr.,* 2011, vol. 67, 271-281
- EVANS ; MURSHUDOV. *Acta Crystallogr. D Biol. Crystallogr.,* 2013, vol. 69, 1204-1214
- MCCOY. *J. Appl. Crystallogr.,* 2007, vol. 40, 658-674
- WINN. *Acta Crystallogr. D Biol. Crystallogr.,* 2011, vol. 67, 235-242
- EMSLEY ; COWTAN. *Acta Crystallogr. D Biol. Crystallogr.,* 2004, vol. 60, 2126-2132
- MURSHUDOV ; VAGIN ; DODSON. *Acta Crystallogr. D Biol. Crystallogr.,* 1997, vol. 53, 240-255
- ADAMS. *Acta Crystallogr. D Biol. Crystallogr.,* 2010, vol. 66, 213-221
- JOOSTEN. *IUCrJ,* 2014, vol. 1, 213-220
- KANTARDJIEFF ; RUPP. *Protein Sci. Publ. Protein Soc.,* 2003, vol. 12, 1865-1871
- KAVAN ; MAN. *Hydrog. Exch. Mass Spectrom.,* 2011, vol. 302, 53-58
- GORDON et al. *Nucleic Acids Res,* 2005, vol. 33, W368-W371
- DOERR et al. *J. Chem. Theory Comput.,* 2016, vol. 12, 1845-1852
- HARVEY ; GIUPPONI ; FABRITIIS. *J. Chem. Theory Comput.,* 2009, vol. 5, 1632-1639
- HOPKINS et al. *J. Chem. Theory Comput.,* 2015, vol. 11, 1864-1874
- HARVEY ; DE FABRITIIS. *J. Chem. Theory Comput.,* 2009, vol. 5, 2371-2377
- NARITOMI ; FUCHIGAMI. *J. Chem. Phys.,* 2011, vol. 134, 065101
- WATERHOUSE et al. *Bioinformatics,* 2009, vol. 25, 1189-1191
- NOTREDAME ; HIGGINS ; HERINGA. *J. Mol. Biol.,* 2000, vol. 302, 205-217
- KABSCH. *Acta Crystallogr. D Biol. Crystallogr.,* 2010, vol. 66, 125-132
- MCCOY et al. *J. Appl. Crystallogr.,* 2007, vol. 40, 658-674
- ADAMS et al. *Methods Struct. Proteomics.,* 2011, vol. 55, 94-106
- EMSLEY et al. *Acta Crystallogr. D Biol. Crystallogr.,* 2010, vol. 66, 486-501
- CHEN et al. *Acta Crystallogr. D Biol. Crystallogr.,* 2010, vol. 66, 12-21